(19) Europäisches Patentamt
European Patent Office
Office européen des brevets

(11) Publication number: **0 147 044**
**B1**

(12) **EUROPEAN PATENT SPECIFICATION**

(45) Date of publication of patent specification: **25.04.90**

(21) Application number: **84307781.9**

(22) Date of filing: **09.11.84**

(51) Int. Cl.[5]: **C 07 D 307/79,**
C 07 D 405/12,
C 07 D 451/04, A 61 K 31/34
// C07C69/84

(54) **Benzofurancarboxamides, process for their preparation and pharmaceutical preparations containing them.**

(30) Priority: **22.12.83 US 564641**

(43) Date of publication of application:
**03.07.85 Bulletin 85/27**

(45) Publication of the grant of the patent:
**25.04.90 Bulletin 90/17**

(84) Designated Contracting States:
**AT BE CH DE FR GB IT LI LU NL SE**

(56) References cited:
**EP-A-0 124 783**
**WO-A-84/03281**
**FR-A-2 325 370**
**FR-A-2 396 757**
**US-A-3 342 826**

(73) Proprietor: **ADRIA LABORATORIES INC.**
**P.O. Box 16529**
**Columbus, Ohio 43216-6529 (US)**

(72) Inventor: **Lednicer, Daniel**
**3252 Leesville Way**
**Dublin Ohio 43017 (US)**
Inventor: **Sun, Jung-Hui**
**c/o Adria Laboratories, Inc. P.O. Box 16529**
**Columbus Ohio 43216 (US)**

(74) Representative: **Bankes, Stephen Charles Digby et al**
**BARON & WARREN 18 South End Kensington**
**London W8 5BU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 147 044 B1

## Description

The present invention relates to a novel group of benzamides which are useful as antiemtic and/or antipsychotic agents. More particularly, it relates to a novel group of benzofuran-7-carboxamides.

There has been little treatment in the literature of the relationship between structure and activity of antiemetic and antipsychotic agents. A large number of chemically quite diverse compounds have been used particularly as antiemetic agents. This is due to the fact that there is more than one physiological cause for these disorders.

U.S. Patent No. 3,342,826 discloses a number of benzamide derivatives which are useful antipsychotic and antiemetic agents. Among the compounds disclosed in the patent is sulpiride, 2-methoxy-N-(1-ethyl-2-pyrrolidinylmethyl)-5-sulfamoyl benzamide. U.S. Patent 3,177,252 to Thominet discloses metoclopramide, 4-amino-5-chloro-N-[(2-diethylamino)ethyl]-2-methoxybenzamide, which has been used clinically to prevent emesis.

Benzamide derivatives function as antiemetic and antipsychotic agents by blocking dopamine receptors in the brain. The administration of sulpiride, metoclopramide, and other prior benzamide derivatives is accompanied by several undesirable side effects including an increase in the release of prolactin and extrapyramidal side effects. It is believed that in blocking certain dopamine receptors benzamide derivatives prevent the secretion of prolactin inhibiting factor or PIF, which regulates the release of prolactin. As a result, there is an elevation in prolactin levels and mammary hypertrophy in the form of breast engorgement, galactorrhea, and amenorrhea results.

Thus, there is a need for effective antiemetic and antipsychotic agents, and particularly, for antiemetic and antipsychotic agents which are free from the aforementioned side effects.

FR—A—2325370 discloses 2-nitro -3 phenyl benzo(b) furan carboxylic acid derivatives which have been found to exhibit anti-inflammatory activity.

EP—A—0124783 discloses 5-Sulphamoyl-benzo (b) furan-7-carboxamides useful in the treatment of psychosomatic diseases, psychic disturbances or other mental disorders.

WO-A-84/03281 discloses *inter alia* 2,3-dihydrobenzo (b) furan carboxamides wherein the amide group includes a bicyclic ring structure, the nitrogen atom of the amide being common to both rings.

The principal object of the present invention is to provide benzofurancarboxamides which are useful as antiemetic and/or antipsychotic agents and pharmaceutical preparations containing the same.

In the case of benzamide derivatives, it appears that the 2-methoxy group located adjacent to the carboxamide linkage in both sulpiride and metoclopramide plays an important role in providing good antipsychotic and antiemetic activity. The compounds of the present invention are characterized in that the oxygen atom in the benzofuran ring is adjacent the carboxamide linkage.

The compounds of the present invention are represented by the general formula (I):

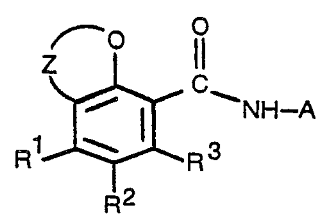

I

wherein Z represents the atoms necessary to complete an unsubstituted or alkyl substituted benzo(b) furan or a dihydrobenzo(b)furan ring; $R^1$, $R^2$ and $R^3$ ring may be the same or different and represent a hydrogen atom, an alkyl group having 1 to 6 carbon atoms, an alkoxy group having 1 to 6 carbon atoms, an amino group, an alkyl substituted amino group, an acylamido group having 1 to 6 carbon atoms, a sulfonamido group having up to 6 carbon atoms and being attached to the benzo(b)furan system by the N-atom, a halogen atom or a nitro group; A represents an aminoalkyl group which contains the atomic sequence:

—C—C—N

In some embodiments the nitrogen atom of the aminoalkyl group forms part of a pyrrolidinyl ring. Typical examples of the alkylamino group represented by A in formula (I) are:

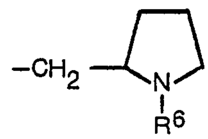

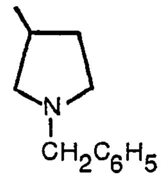

$-CH_2CH_2NR^7R^8$

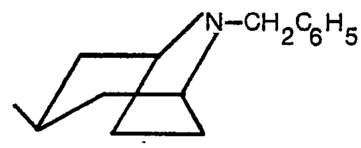

wherein $R^6$, $R^7$ and $R^8$ each represent a hydrogen atom or a lower alkyl group containing 1 to 6 carbon atoms.

The present invention is more specifically directed to benzo[b]furan and dihydrobenzo[b]furan-carboxamides represented by the formula II and III wherein $R^1$, $R^2$ and $R^3$ are defined as above and $R^9$ is a hydrogen atom or an alkyl group having 1 to 3 carbon atoms and to pharmaceutical preparations containing the same.

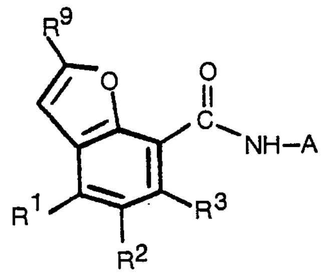

II

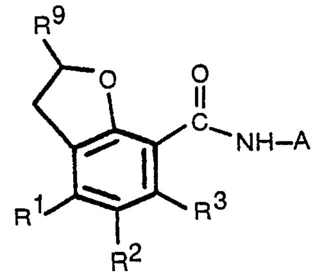

III

The present invention is still more specifically directed to compounds of the formula I, II or III wherein $R^2$ is a lower alkyl group and to pharmaceutical compositions containing the same.

With reference to formulae I, II, and III above, in more detail Z represents the atoms necessary to complete a benzo[b]furan or a dihydrobenzo[b]furan ring which may be substituted in the 2- or 3-position by a lower alkyl group such as a methyl group or an ethyl group or may be unsubstituted. Thus, where Z represents the atoms necessary to form a dihydrobenzo[b]furan ring, Z may be a $CnH_{2n}$ group where n is 2 to 4 such as $—CH_2CH_2—$, $CH_2CH(CH_3)—$, $—CH_2C(CH_3)_2—$, $—CH(CH_3)CH_2—$, $—CH_2CH(C_2H_5)—$, etc. Where Z represents the atoms necessary to form a benzofuran ring, Z may represent $—CH{=}CH—$, $—CH{=}CCH_3—$, or $—CH_3C{=}CH—$.

Representative examples of the alkyl group represented by $R^1$, $R^2$ or $R^3$ include lower alkyl groups having 1 to 6 carbon atoms such as methyl, ethyl, n-propyl, i-propyl, and t-butyl groups.

Representative examples of the halogen atoms represented by $R^1$, $R^2$ and $R^3$ include fluorine, chlorine, bromine and iodine atoms.

The amino group represented by $R^1$, $R^2$ or $R^3$ may be an unsubstituted amino group or a substituted amino group of the formula $—NR^4R^5$ wherein $R^4$ and $R^5$ may be the same or different and selected from a hydrogen atom or an alkyl group containing 1 to 6 carbon atoms such as methyl, ethyl, n-propyl, etc. Otherwise, the amino group can be a substituted amino group such as an acylamido or a sulfonamido group of the formula $—NHCOR^4$ and $—NHSO_2R^4$ wherein $R^4$ is defined as above.

Representative examples of the alkoxy groups for $R^1$, $R^2$ and $R^3$ include alkoxy groups having 1 to 6 carbon atoms such as methoxy, ethoxy, and propoxy.

Of the compounds represented by the formula I, II and III, those which appear to be particularly preferred are compounds in which $R^2$ is an alkyl group and A is a 2-(N-ethyl)pyrrolidinylmethyl group.

The compounds of the present invention exhibit antipsychotic and antiemetic activity in *in vivo* screening tests as shown below. The compounds can be compounded with a suitable pharmaceutical carrier for parenteral or oral administration. In most cases the compounds are useful in the form of the acid addition salt, e.g., the hydrochloride, phosphate, fumarate, citrate, tartarate. Therapeutically effective dosages will lie in the range of about 0.01 to 10 mg/kg.

The compounds of the present invention are conveniently prepared by condensing benzo[b]furan-7-carboxylic or dihydrobenzo[b]furan-7-carboxylic acid chlorides or esters with appropriate amines and recovering the carboxamides as acid salts. Hereinbelow, four synthetic approaches to the carboxamides are described.

The synthesis of the benzofuran-7-carboxylic acid esters, which are convertible to the acid chloride to produce benzofurancarboxamides in accordance with the present invention proceeds along Reaction Scheme I:

REACTION SCHEME I

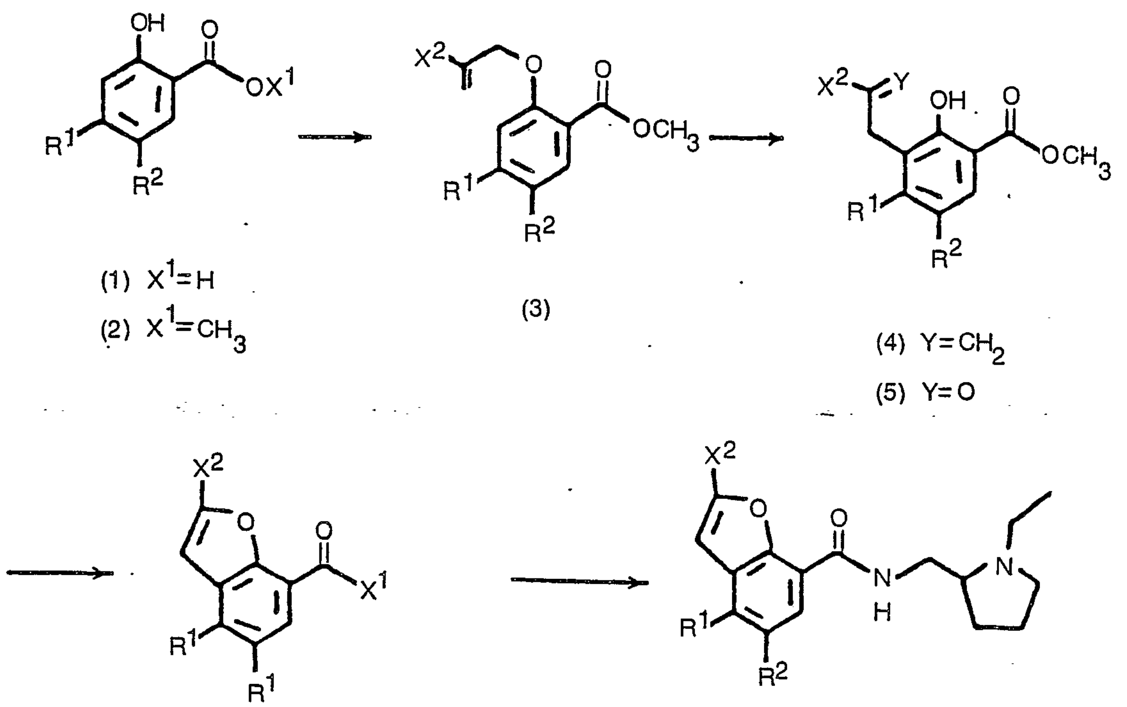

(1) $X^1=H$
(2) $X^1=CH_3$

(3)

(4) $Y=CH_2$
(5) $Y=O$

(6) $X^1=OCH_3$
(7) $X^1=OH$
(8) $X^1=Cl$

(9)

The compounds of the present invention can be prepared from commercially available substituted or unsubstituted salicylic acids. In accordance with Reaction Scheme I, 2-, 3-, 4-, 5- or 6-substituted or unsubstituted benzofuran-7-carboxamides can be synthesized from salicylic acid (1) by treating the acid with methanol in the presence of sulfuric acid, as a catalyst, to produce the methyl ester (2). The ester (2) can be alkylated to give the intermediate (3) in over 90% yield by nucleophilic substitution of the phenoxide ion with an allyl halide in acetone containing potassium carbonate. The 2-unsubstituted derivatives are prepared by substitution with allyl bromide. The 2- or 3-alkyl substituted derivatives are prepared by reacting with 3-chloro-2-alkyl propene or 3-chloro-3-alkyl propene. The products are clean and can be used for subsequent reaction without purification.

By heating the intermediate 3 at about 200°C under argon either without any solvent (Method A) or with N,N-dimethylaniline (Method B), a Claisen rearrangement takes place resulting in the compound 4. Table I below summarizes the Claisen rearrangement conditions for obtaining eight intermediates (Compounds 4a-4h) by this method and provides the relevant analytical data. Attempts to distill compounds 4f and 4g led to mixtures possibly due to production of para isomers by further rearrangement. Chromatography of the reaction mixture 4g gave the desired compound 4g along with the p-isomer in 3.7% yield. Column chromatography of compound 4f led to isolation of four products including compound 4f.

TABLE I

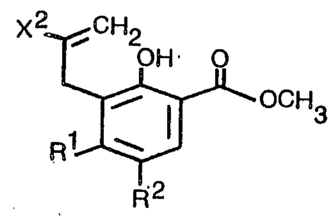

| Compounds | $R^1$ | $R^2$ | $X^2$ | Method | Claisen Rearrangement temp (°C) | Reaction time (hr) | Yield (%) | bp (mm) | Formula (Calc'd) |
|---|---|---|---|---|---|---|---|---|---|
| 4a | H | H | H | A | 195—200 | 16 | 92.5 | 80—95 (0.02) | $C_{11}H_{12}O_3$ |
| 4b | H | Br | H | A | 190 | 20 | 89.4 | 110—126 (0.25—0.5) | $C_{11}H_{11}BrO_3$ |
| 4c | H | Cl | H | A | 200 | 20 | 88.5 | 130—136 (3.5) | $C_{11}H_{11}ClO_3$ |
| 4d | H | F | H | A | 190—200 | 20 | 88.0 | 83—89 (1.0) | $C_{11}H_{11}FO_3$ |
| 4e | H | $OCH_3$ | H | A | 205 | 64 | 87.0 | 142—144 (3.0) | $C_{12}H_{14}O_4$ |
| 4f | Cl | H | H | A | 200 | 38 | 32.9 | 117—121 | $C_{11}H_{11}ClO_3$ |
| | | | | B | 190—205 | 20 | 71.1 | | |
| 4g | $OCH_3$ | H | H | B | 190—205 | 18 | 59.5 | 55—58 (mp) | $C_{12}H_{14}O_4$ |
| 4h | H | H | $CH_3$ | A | 200 | 18 | 90.8 | 102—108 (3.0) | $C_{12}H_{14}O_3$ |

The intermediate 4 can be oxidized by reaction with $OsO_4/NaIO_4$ in ethyl ether and water at room temperature to produce the corresponding aldehydes or ketones (5) in very good yields. Due to the instability of the aldehydes and ketones (5), they are carried to the next step (acid-catalyzed cyclization) without purification.

Acid catalysts are employed for cyclization of the aldehydes or ketones to produce compound (6). The acid catalysts of choice are either Amberlyst-XN1010 and trifluoroacetic acid (TFA). TFA is more preferred because it requires a shorter reaction time and generates fewer by-products. Using either catalyst, the phenolic hydroxy group attacks the aldehyde to form a hemiacetal which loses water and results in the benzofuran ester (6).

Table II below provides the analytical data for six benzofuran-7-carboxylic acid esters produced by Reaction Scheme I.

TABLE II

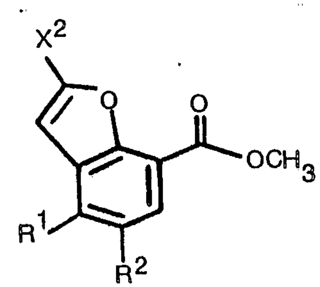

| Compounds | $R^1$ | $R^2$ | $X^2$ | Cyclization Catalysts | Chromatographic solvent | Recryst. solvent | Yield (%) | mp (°C) | Analytical Analysis* |
|---|---|---|---|---|---|---|---|---|---|
| 6a | H | H | H | Amberlyst XN1010 | Hexane (100%) to EtOAc/hexane (7.5/100) | — | 50.7 | liquid | $C_{10}H_8O_3$ |
| 6b | H | Br | H | Amberlyst XN1010 | Hexane $CH_2Cl_2$ (1/1 to 1/3) | — | 62.4 | 156—158 | $C_{10}H_7BrO_3$ |
| 6c | H | Cl | H | Amberlyst XN1010 | $CH_2Cl_2$ | $CH_2Cl_2$/hexane | 21.0 | 144—145 | $C_{10}H_7ClO_3$ |
| | | | | TFA/ | $CH_2Cl_2$ | — | 63.2 | | |
| 6d | H | F | H | TFA/rt | $CH_2Cl_2$ | $Et_2O$/hexane | 57.8 | 100—102 | $C_{10}H_7FO_3$ |
| 6f | Cl | H | H | TFA/rt | $CH_2Cl_2$ | — | 32.3 | 105—106 | $C_{10}H_7ClO_3$ |
| 6h | H | H | $CH_3$ | Amberlyst XN1010 | EtOAc/hexane (1/9) | — | 54.0 | liquid | $C_{11}H_{10}O_3$ |

*Actual values for elemental combustion analysis were within 0.4% of calculated values. Nitrogen containing compounds were analyzed for C, H, N; others for C, H only.

The ester (6) can be easily converted to the acid (7) and from the acid (7) to the acid chloride (8). Reaction of acid chloride (8) with an amine such as 2-aminomethyl-1-ethylpyrrolidine as illustrated provides the 7-carboxamide (9) without difficulty.

Table III below summarizes the analytical data for six benzo[b]furan-7-carboxamides in accordance with the present invention produced by Reaction Scheme I.

TABLE III

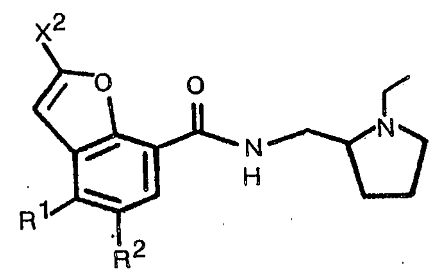

| Compounds | $R^1$ | $R^2$ | $X^2$ | Salt | Recryst Solvent | Yield (%) | mp (°C) | Analytical Analysis* |
|---|---|---|---|---|---|---|---|---|
| 9a | H | H | H | $1.5C_4H_4O_4$** | 2-butanone | 38.9 | 131—132.5 | $C_{16}H_{20}N_2O_2$ $.1.5C_4H_4O_4$ |
| 9b | H | Br | H | — | EtOAc/hexane | 49.7 | 81—83 | $C_{16}H_{19}BrN_2O_2$ |
| 9c | H | Cl | H | $0.5C_4H_4O_4$ | 2-butanone | 44.3 | 158—159 | $C_{16}H_{19}ClN_2O_2$ $.0.5C_4H_4O_4$ |
| | | | | $C_4H_4O_4$ | 2-butanone | 64.9 | 126.5—128.5 | $C_{16}H_{19}ClN_2O_2$ $C_4H_4O_4$ |
| 9d | H | F | H | $C_4H_4O_4$ | 2-butanone | 64.6 | 140—142 | $C_{16}H_{19}FN_2O_2$ $.C_4H_4O_4$ |
| 9f | Cl | H | H | $1.25C_4H_4O_4$ | 2-butanone | 30.4 | 115—119 | $C_{16}H_{19}ClN_2O_2$ $1.25C_4H_4O_4$ |
| 9H | H | H | $CH_3$ | — | — | 51.1 | liquid | $C_{17}H_{22}N_2O_2$ |

*Actual values for elemental combustion analysis were within 0.4% of calculated values. Nitrogen containing compounds were analyzed for C, H, N; others for C, H only.
**$C_4H_4O_4$ = fumarate

Reaction Scheme I above is illustrated in more detail in Synthesis Example 1 below by reference to the synthesis of 5-chloro-N-(1-ethyl-2-pyrrolidinylmethyl)benzo[b]furan-7-carboxamide fumarate (Compound 9c).

Reaction Schemes II—IV below are alternative syntheses for dihydrobenzo[b]furan-7-carboxamides.

Reaction Scheme II which is particularly useful in synthesizing the 2-alkyl-2,3-dihydrobenzo[b]furan derivatives proceeds from the Claisen rearrangement product (4) of Reaction Scheme (I) as follows:

REACTION SCHEME II

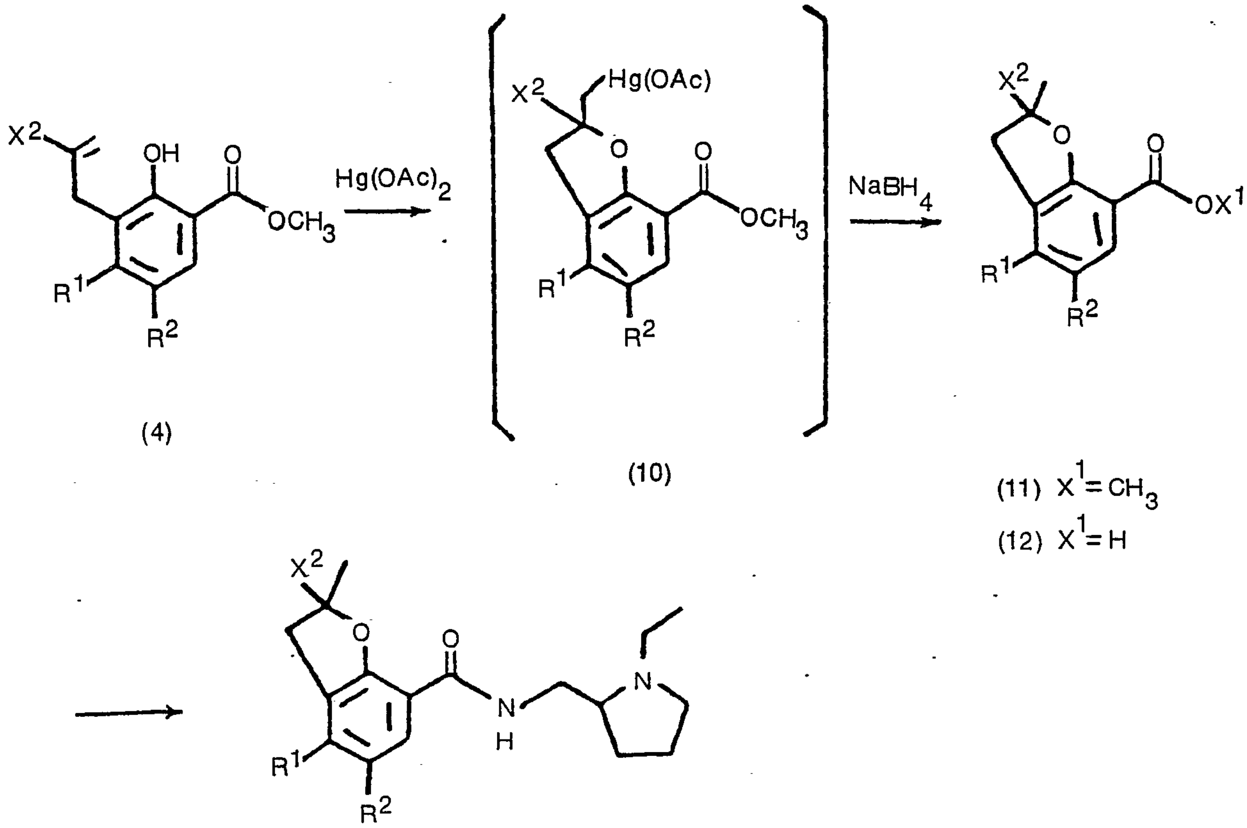


(4)

(10)

(11) $X^1 = CH_3$

(12) $X^1 = H$

(13)

The intermediate (10) is obtained by reacting the product (4) with mercuric acetate in dry THF in the dark.

Reduction of intermediate (10) with sodium borohydride in 3N NaOH gives, upon workup by column chromatography, the dihydrobenzo[b]furan-7-carboxylic ester (11). The ester can be converted to the corresponding acid chloride and reacted with an appropriate amine to give dihydrobenzo[b]furans such as compound (13) as previously outlined. Table IV below provides the analytical data for dihydrobenzo[b]furan esters (11) obtained by Reaction Scheme II.

TABLE IV

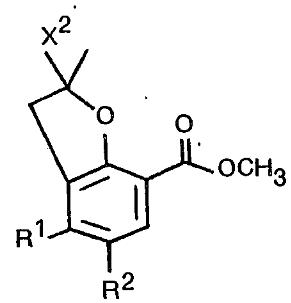

| Compounds | $R^1$ | $R^2$ | $X^2$ | Chromatographic solvent | Recryst. solvent | Yield (%) | mp (°C) | Analytical Analysis* |
|---|---|---|---|---|---|---|---|---|
| 11a | H | H | H | EtOAc/hexane (0/100 to 20/100) | — | 54.0 | oil | $C_{11}H_{12}O_3$ |
| 11b | H | Br | H | EtOAc/hexane (0/100 to 25/100) | Hexane | 47.2 | 67—68 | $C_{11}H_{11}BrO_3$ |
| 11e | H | $OCH_3$ | H | — | Hexane | 68.3 | 75—77 | $C_{12}H_{14}O_4$ |
| 11f | Cl | H | H | — | Hexane | 71.9 | 77—80 | $C_{11}H_{11}ClO_3$ |
| 11g | $OCH_3$ | H | H | $CH_2Cl_2$ | — | 63.2 | 6.55—68.5 | $C_{12}H_{14}O_3$ |
| 11h | H | H | $CH_3$ | EtOAc/hexane (0/100 to 10/100) | — | 53.0 | oil | $C_{12}H_{14}O_3$ |

*Actual values for elemental combustion analysis were within 0.4% of calculated values. Nitrogen containing compounds were analyzed for C, H, N; others for C, H only.

Substituents can be introduced into the benzene ring by selecting the appropriate substituted salicyclic acid as a starting material or by reacting the benzofuran carboxylic acid with the appropriate electrophilic reagent. For example, chlorination of the 4-chloro derivative (12f) with sulfuryl chloride at room temperature furnishes the 4,5-dichloro derivative (12i) (Table V) in 54% yield. Nitration of compound (12f) in refluxing $HNO_3$/TFA gives the 4-chloro-5-nitro derivative (12j) in moderate yield. In general it is desired to perform the electrophilic substitutions before condensing the amine to avoid hydrolysis of the amine during the substitution. Other modifications can be performed after condensation and are discussed below.

Table V below provides the reaction conditions for hydrolysis/substitution of the esters (11) with the yield and melting point for seven dihydrobenzofuran-7-carboxylic acid derivatives produced by following Reaction Scheme II. The carboxamides (13) are prepared by reaction of the acid chlorides or the esters (11) by heating with the appropriate amine, which in the case of compound (13) is 2-aminomethyl-1-ethylpyrrolidine. In most cases, the carboxamides are most conveniently isolated as their fumarate salts.

Reaction Scheme II is illustrated in more detail in Synthesis Example 2 for the synthesis of 4 - chloro - N - (1 - ethyl - 2 - pyrrolidinylmethyl) - 2 - methyl - 2,3 - dihydrobenzo[b]furan - 7 - carboxamide fumarate (compound 13f). Table VI below summarizes the analytical data of several dihydrobenzofuran-7-carboxamides in accordance with the present invention.

TABLE V

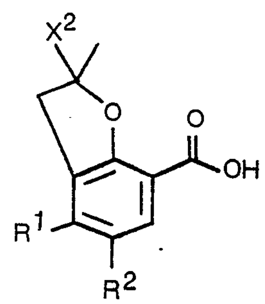

| Compounds | $R^1$ | $R^2$ | $X^2$ | Reaction Conditions | Recryst. Solvent | Yield (%) | mp (°C) |
|---|---|---|---|---|---|---|---|
| 12a | H | H | H | 10% NaOH | — | 96.5 | oil |
| 12b | H | Br | H | $KOH/CH_3OH/H_2O$ | — | 95.3 | 206—209 |
| 12e | H | $OCH_3$ | H | $KOH/CH_3OH/H_2O$ | — | 95.6 | 122—124 |
| 12f | Cl | H | H | $KOH/CH_3OH/H_2O$ | — | 99.0 | 210—213 |
| 12g | $OCH_3$ | H | H | $KOH/CH_3OH/H_2O$ | — | 96.5 | 209—211 |
| 12i | Cl | Cl | H | $SO_2Cl_2$ | EtOAc | 54.3 | 204—207 |
| 12j | Cl | $NO_2$ | H | $HNO_3$/TFA | EtOAc | 34.8 | 193—197 |

TABLE VI

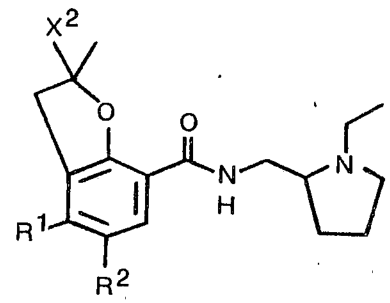

| Compounds | $R^1$ | $R^2$ | $X^2$ | Methods | Salts | Recryst. Solvent | Yield (%) | mp (°C) | Analytical Analysis* |
|---|---|---|---|---|---|---|---|---|---|
| 13a | H | H | H | A | — | — | 76.5 | oil | $C_{17}H_{24}N_2O_2$ |
| 13b | H | Br | H | B | HCl | $CH_2Cl_2$/hexane | 53.2 | 168—170 | $C_{17}H_{23}BrN_2O_2.HCl$ |
| 13e | H | $OCH_3$ | H | B | $C_4H_4O_4$** | — | 62.8 | 143.5—145 | $C_{18}H_{26}N_2O_3.C_4H_4O_4$ |
| 13f | Cl | H | H | A | $C_4H_4O_4$ | 2-butanone | 45.2 | 163—164.5 | $C_{17}H_{23}ClN_2O_2.C_4H_4O_4$ |
| 13g | $OCH_3$ | H | H | A | $C_4H_4O_4$ | 2-butanone | 33.3 | 144—147 | $C_{18}H_{26}N_2O_3.C_4H_4O_4$ |
| 13h | H | H | $CH_3$ | B | $C_4H_4O_4$ | EtOH/acetone | 42.3 | 158—160 | $C_{18}H_{26}N_2O_2.C_4H_4O_4$ |
| 13i | Cl | Cl | H | A | $C_4H_4O_4$ | 2-butanone | 37.3 | 157—159 | $C_{17}H_{22}ClN_2O_2.C_4H_4O_4$ |
| 13j | Cl | $NO_2$ | H | A | — | — | 87.9 | solid | $C_{17}H_{22}ClN_3O_4$ |
| 13k | Cl | $NH_2$ | H | Pd/C | $C_4H_4O_4$ | acetone | 31.2 | 165—168 | $C_{17}H_{24}ClN_3O_2.C_4H_4O_4$ |

Actual values for elemental combustion analysis were within 0.4% of calculated values. Nitrogen containing compounds were analyzed for C, H, N; others for C, H only.
*$C_4H_4O_4$ = fumarate

2,3-Dihydrobenzo[b]furan-7-carboxylic acids (15) can be prepared according to Reaction Scheme III by lithiation of 2,3-dihydrobenzo[b]furan (14) with n-butyl lithium in TMEDA/hexane at room temperature, followed by quenching with dry ice and acidifying with concentrated HCl.

REACTION SCHEME III

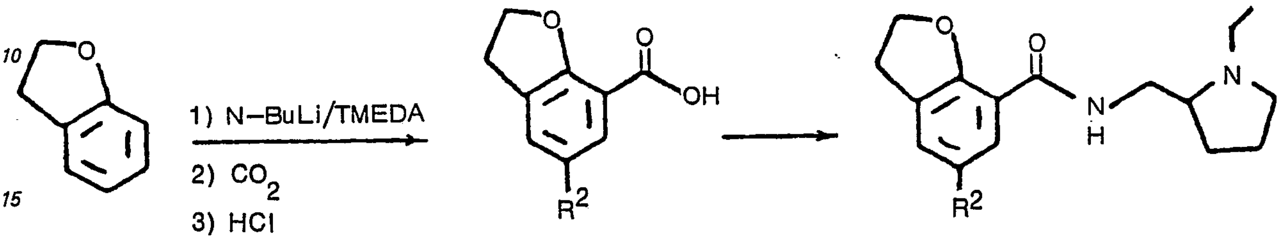


(14)

(15a) $R^2$=H
(15b) $R^2$=Br
(15c) $R^2$=Cl
(15k) $R^2$=$NO_2$

(16a) $R^2$=H
(16b) $R^2$=Br
(16c) $R^2$=Cl
(16k) $R^2$=$NO_2$

(16k)

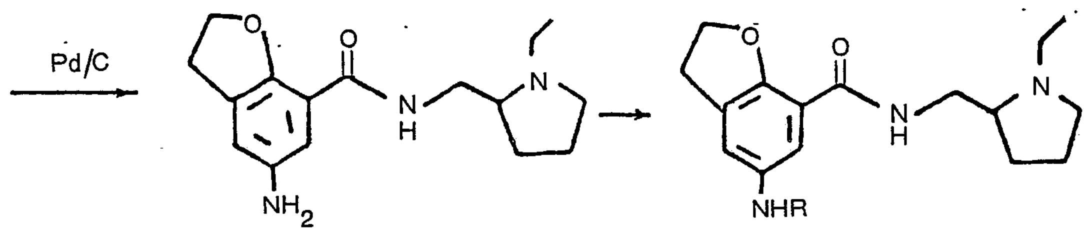


(16l)

(16m) R=$COCH_3$
(16n) R=$SO_2CH_3$

The acid (15a) serves as a starting material for other 5-substituted derivatives. Bromination of 15a with bromine in acetic acid containing a trace amount of iron yields the 5-bromo derivative (15b). Chlorination of (15a) with sulfuryl chloride at room temperature affords the 5-chloro derivative (15c). Nitration of (15a) carried out in either $HNO_3$/TFA at 0°C to room temperature or $HNO_3$ in acetic acid at 70°C yields the 5-nitro derivative (15k).

Other modifications can be performed after condensation with the amine. In particular, the 5-nitro derivative can be catalytically reduced to produce the 5-amino derivative. Acetylation or sulfonylation of the 5-amino compound produces the corresponding acylamide and sulfonamide. These compounds together with their melting point and analytical data are shown in Table VII below. The reactions are illustrated in Synthesis Examples 5, 6 and 7 below.

TABLE VII

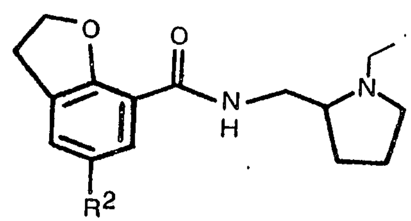

| Compounds | $R^2$ | Salts | Recryst. Solvent | Yield (%) | mp (°C) | Analytical Analysis* |
|---|---|---|---|---|---|---|
| 16a | H | $1.5C_4H_4O_4$** | 2-butanone | 49.7 | 151—152.5 | $C_{16}H_{22}N_2O_2.1.5C_4H_4O_4$ |
| 16b | Br | $C_4H_4O_4$ | 2-butanone | 41.1 | 160—161.5 | $C_{16}H_{21}BrN_2O_2.C_4H_4O_4$ |
| 16c | Cl | $1.5C_4H_4O_4$ | 2-butanone | 42.9 | 149—150 | $C_{16}H_{21}ClN_2O_2.1.5C_4H_4O_4$ |
| 16k | $NO_2$ | HCl | 2-butanone | 56.2 | 206.5—208.5 | $C_{16}H_{21}N_3O_4.HCl$ |
| 16l | $NH_2$ | $C_4H_4O_4.0.5H_2O$ | 2-butanone | 41.4 | 98—101 | $C_{16}H_{23}N_3O_2.C_4H_4O_4.0.5H_2O$ |
| 16m | $NHCOCH_3$ | $C_4H_4O_4$ | EtOH | 63.4 | 179—181 | $C_{18}H_{25}N_3O_3.C_4H_4O_4$ |
| 16n | $NHSO_2CH_3$ | — | EtOH | 74.6 | 180—182 | $C_{17}H_{25}N_3O_4S$ |

*Actual values for elemental combustion analysis were within 0.4% of calculated values. Nitrogen containing compounds were analyzed for C, H, N; others for C, H only.
**$C_4H_4O_4$ = fumarate

Reaction Scheme III is illustrated in more detail by reference to the synthesis of 2,3-dihydrobenzo[b]furan-7-carboxylic acid (15a) and the 5-bromo and 5-nitro derivatives thereof in Synthesis Example 3 below.

Dihydrobenzofuran-7-carboxamides can also be obtained through the following Reaction Scheme IV from 2,6-dibromophenols

REACTION SCHEME IV

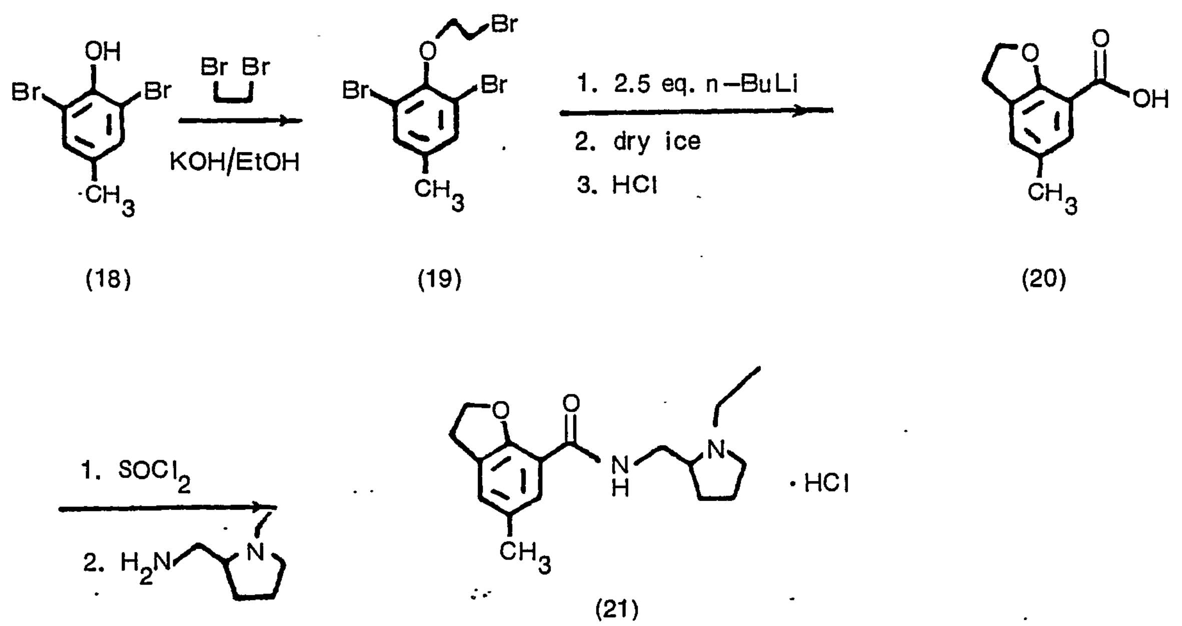


(18) (19) (20)

(21)

Reaction Scheme IV proceeds via alkylation of a 2,6-dibromophenol (18) by reaction with 1,2-dibromoethane to provide the phenyloxyethyl bromide (19). By lithiating the bromide (19) using n-butyl lithium, quenching in dry ice and acidifying 2,3-dihydrobenzo[b]furan-7-carboxylic acid (20) is obtained. This acid is then converted to the acid chloride and this is reacted with the appropriate amine to give the carboxamide; alternatively, the acid can be first converted to the ester. Reaction of this with the amine leads to the carboxamides.

Reaction Scheme IV is illustrated in more detail in Synthesis Example 4 below wherein 5 - methyl - N - (1 - ethyl - 2 - pyrrolidinylmethyl) - 2,3 - dihydrobenzo[b]furan - 7 - carboxamide hydrochloride (21) is prepared by this reaction route.

Hereinbelow the foregoing syntheses are illustrated in more detail by the following non-limiting Synthesis Examples.

Synthesis Example 1

a. Methyl 5-chloro-2-hydroxybenzoate *(2c)*

A mixture of 5-chloro-2-hydroxybenzoic acid (100 g, 0.57 mol), 20 ml of conc. $H_2SO_4$ and 200 ml of dry methanol was heated under reflux for 22 hours. An additional 5 ml of conc. $H_2SO_4$ was then added and the solution heated for an additional 24 hours. The solvent was evaporated under reduced pressure and the resulting residue poured into aqueous saturated $Na_2CO_3$ and then extracted with $CH_2Cl_2$ (2 × 400 ml). The organic layers were combined, dried over anhydrous $Na_2SO_4$, and evaporated to give the desired compound as a white solid (100.5 g, 94.8% yield), mp 44—46°C. IR (nujol) 1680 (ester) $cm^{-1}$. NMR ($CDCl_3$) δ 10.60 (s, 1H, OH), 7.73 (d, 1H, $J_{BC}$=3Hz $H_C$), 7.33 (d of d, 1H, $J_{AB}$=9Hz, $H_B$), 6.87 (d, 1H, $H_A$), and 3.92 (s, 3H, $CH_3$).

b. Preparation of methyl 2-allyloxy-5-chlorobenzoate *(3c)*

A mixture of methyl 5-chloro-2-hydroxybenzoate *(2c)* (45.0 g, 0.24 mol), allyl bromide (62.6 ml, 0.75 mol) and ground potassium carbonate (66.67 g, 0.48 mol) in 750 ml of dry acetone was heated under reflux for 2½ hours. The inorganic salt was removed by filtration, and the solvent and excess allyl bromide in the filtrate was evaporated to dryness to yield compound *3c* quantitatively as a white solid, mp 25—26°C. IR (nujol) 1738 (ester) $cm^{-1}$. NMR ($CDCl_3$) δ 7.63 (d, 1H, $J_{BC}$=3Hz, $H_C$), 7.27 (d of d, 1H, $J_{AB}$=9 Hz, $H_B$), 6.77 (d. 1H, $H_A$), 6.30—6.47 (m, 3H, olefinic protons) and 3.87 (s, 3H, $CH_3$).

c. Preparation of methyl 5-chloro-2-hydroxy-3-(2-propenyl)benzoate (*4c*)

Methyl 2-allyloxy-5-chlorobenzoate (*3c*) (76.66 g, 0.34 mol) was heated at 200°C without solvent under argon for 20 hours. The dark brown liquid was distilled to give 67.9 g (88.5%) of product *4c* as a clear liquid, b.p. 130—136°C (3.4 torr). IR (neat) 1675 (ester) $cm^{-1}$. NMR ($CDCl_3$) δ 10.50 (s, 1H, OH), 7.20 (d, 1H, J=3Hz, aromatic proton), 7.13 (d, 1H, aromatic proton), 6.23—4.80 (m, 3H, olefinic protons), 3.90 (s, 3H, $CH_3$), and 3.32 (d, 2H, J=6 Hz, $CH_2$).

d. Preparation of 3-carbomethoxy-5-chloro-2-hydroxyphenylacetaldehyde (*5c*)

Osmium tetroxide (1.0 g, 3.9 mmol) was added to a mixture of methyl 5-chloro-2-hydroxy-3-(2-propenyl)benzoate (*4c*) (22.7 g, 0.1 mol), ether (400 ml), and water (400 ml). After stirring at room temperature for 5 minutes, sodium periodate (47.06 g, 0.22 mol) was added in portions. The mixture was stirred at room temperature for another 21 hours, and then poured into 800 ml of water. The layers were separated and the aqueous layer extracted with ethyl ether (2 × 400 ml). The organic layer were combined, dried over anhydrous $Na_2SO_4$ and then filtered through a column of Florisil (85 g) to remove inorganic impurity. Evaporation of the ether yielded *5c* quantitatively as a brown solid, m.p. 49—59°C, which appeared hygroscopic. The crude product was used for the next reaction without purification. NMR ($CDCl_3$) δ 10.90 (s, 1H, OH), 9.67 (t, 1H, J=1.5Hz, CHO), 7.70 (d, 1H, J=3Hz, aromatic proton), 7.23 (d, 1H, aromatic proton), 3.97 (s, 3H, $CH_3$) and 3.70 (d, 2H, $CH_2$).

e. Preparation of 7-carbomethoxy-5-chlorobenzo[b]furan (*6c*)

A trifluoroacetic acid (25 ml) solution of 3-carbomethoxy-5-chloro-2-hydroxyphenylacetaldehyde (*5c*) (5.20 g, 22.7 mmol) was refluxed for 2 hours under argon, and then poured into saturated $Na_2CO_3$. The resulting precipitate was collected by filtration, and then purified by column chromatography (100% $CH_2Cl_2$) to give *6c* as a cream colored solid (3.02 g, 63.2% yield). Analytical sample was prepared by recrystallization from ethyl ether, mp 144—145°C. IR (nujol) 1710 (ester) $cm^{-1}$. NMR ($CDCl_3$) δ 7.87—6.70 (m, 4H, aromatic protons) and 4.0 (s, 3H, $CH_3$).

Anal. Calcd for $C_{10}H_7ClO_3$: C, 57.03; H, 3.35. Found: C, 57.03, H, 3.48.

f. Preparation of 5-chlorobenzo[b]furan-7-carboxylic acid (*7c*)

A solution of 7-carbomethoxy-5-chlorobenzo[b]furan (*6c*) (3.02 g, 14.3 mmol) containing potassium hydroxide (3.22 g) in 100 ml of aqueous methanol ($CH_3OH/H_2O$=3/2) was stirred at room temperature overnight. The mixture was concentrated under reduced pressure. The residue was acidified with conc. HCl to pH of about 1, cooled in freezer for several hours and filtered to give *7c* as an off-white solid (2.65 g, 94.3%), mp 212—215°C. NMR (DMSO-$d_6$) δ 8.0—6.87 (m, 5H, aromatic protons and OH).

g. Preparation of 5-chloro-N-(1-ethyl-2-pyrrolidinylmethyl)benzo[b]furan-7-carboxamide fumarate (*9c*)

A mixture of 5-chlorobenzo[b]furan-7-carboxylic acid (*7c*) (4.41 g, 22.4 mmol) and thionyl chloride (6.5 ml, 89.1 mmol) in 90 ml of toluene was heated under reflux for 2½ hours. The solvent and excess thionyl chloride were removed under reduced pressure to give the corresponding 7-carboxylic acid chloride (*8c*) as a yellow solid. The acid chloride was then dissolved in 90 ml of methylene chloride and cooled to 0°C with an ice bath. To this solution was added dropwise 2-aminomethyl-1-ethylpyrrolidine (2.88 g, 22.4 mmol) in 20 ml of methylene chloride. The yellow solution was stirred at ambient temperature overnight, and then poured into 90 ml of 10% NaOH. The layers were separated, and the organic layer dried over anhydrous $Na_2SO_4$ and evaporated to give the desired product *9c* as its free base (6.46 g, 94.0% yield). The carboxamide is conveniently isolated as its fumarate by adding to the free base in 100 ml of absolute ethanol 1 equivalent of fumaric acid. After stirring at room temperature for 1 hour, the solution was reduced to a small volume, this was triturated with acetone, cooled in the freezer briefly, and the solid collected on a filter to give *9c* as an off-white solid (7.64 g, 80.7%). The analytical sample was prepared by recrystallization from 2-butanone, mp 126.5—128.5°C. NMR (DMSO-$d_6$) δ 8.70 (br s, 1H, exchangeable with $D_2O$, NH), 8.0 (d, 1H, J=2Hz, aromatic proton), 7.77 (d, 1H, aromatic proton), 7.57 (d, 1H, aromatic proton) 7.20 (br s, exchangeable with $D_2O$, 2.COOH), 6.93 (d, 1H, aromatic proton), 6.47 (2, 2H, 2CH), 3.70—1.60 (m, 11H, $5CH_2$ and CHN), and 1.20 (t, 3H, J=7Hz, $CH_3$).

Anal. Calcd for $C_{16}H_{19}ClN_2O_2$. $C_4H_4O_4$: C, 56.81; H, 5.48; N, 6.62. Found: C, 57.11; H, 5.52; N, 6.52.

## Synthesis Example 2

a. Preparation of methyl 4-chloro-2-hydroxybenzoate (*2f*)

A mixture of 4-chloro-2-hydroxybenzoic acid (200 g, 1.16 mol) and 40 ml conc. sulfuric acid in 400 ml of methanol was heated under reflux for 20½ hours. The solvent was evaporated under reduced pressure, the residue poured into aqueous saturated $Na_2CO_3$, and then extracted with ethyl ether (3 × 500 ml). The combined organic layers were dried over anhydrous $Na_2SO_4$, and evaporated to give *2f* (191.6 g, 88.6% yield) as an oil. This was used for the next reaction without purification. IR (neat) 3150 (OH), 1728 (C=0), and 1678 (C=0), and 1678 (C=O) $cm^{-1}$. NMR ($CDCl_3$) δ 10.27 (br s, 1H, OH), 7.47 (d, 1H, $J_{BC}$=8 Hz, $H_C$), 6.80 (d, 1H, $J_{AB}$=2 Hz, $H_A$), 6.63 (d of d, 1H, $H_B$), and 3.85 (s, 3H, $CH_3$).

b. Preparation of methyl 2-allyloxy-4-chlorobenzoate (*3f*)

A mixture of methyl 4-chloro-2-hydroxybenzoate (*2f*) (96.3 g, 0.52 mol), allyl bromide (134 ml, 1.55 mol) and ground potassium carbonate (107 g, 0.77 mol) in 1500 ml of dry acetone was heated under reflux for 20 hours. The inorganic salt was removed by filtration, and the solvent and excess allyl bromide evaporated to dryness to give *3f* (117 g, 99.6% yield) as a yellow solid, mp 47—55°C. NMR ($CDCl_3$) δ 7.73 (d, 1H, J=8Hz, aromatic proton meta to Cl), 6.97 (m. 2H, aromatic protons alpha to Cl), 6.40—5.17 (m, 3H, olefinic protons), 4.57 (m, 2H, $CH_2$) and 3.90 (s, 3H, $CH_3$).

c. Preparation of methyl 4-chloro-2-hydroxy-3-(2-propenyl benzoate) (*4f*)

A solution of methyl 2-allyloxy-4-chlorobenzoate (*3f*) (50.6 g, 0.22 mol) in 100 ml of N,N-dimethylaniline was heated under argon in an oil bath at 190—205°C for 20 hours. The solvent was removed by distillation and the residue purified by column chromatography (silica gel, $CH_2Cl_2$, hexane) to give *4f* (36.0 g, 71.1% yield) as a very light oil. IR (nujol) 1665 (C=0) $cm^{-1}$. NMR ($CDCl_3$) δ 11.23 (S, 1H, OH), 7.47 (d, 1H, J=9 Hz, aromatic proton meta to Cl), 6.77 (d, 1H, aromatic proton alpha to Cl), 6.17—4.77 (m, 3H, olefinic protons), 3.87 (s, 3H, $CH_3$) and 3.50 (m, 2H, $CH_2$).

d. Preparation of 7-carbomethoxy-4-chloro-2-methyl-2,3-dihydrobenzo[b]furan (*11f*)

A solution of methyl 4-chloro-2-hydroxy-3-(2-propenyl)benzoate (*4f*) (4.17 g, 18.4 mmol) and mercuric acetate (5.86 g, 18.4 mmol) in 60 ml of THF was heated under reflux for 3 hours, and then cooled to room temperature. To this was added dropwise at 0°C a solution of 0.70 g (18.4 mmol) sodium borohydride in 4.2 ml of 3 N NaOH. When the addition was complete, the mixture was stirred at ambient temperature for an additional 2 hours and then treated with 55 ml saturated $Na_2CO_3$. The precipitated mercury was removed by filtration with the aid of Celite. The organic layer was separated, dried over anhydrous $Na_2SO_4$ and evaporated to give a mixture of product *11f* and starting material (*4f*) (total 3.89 g). The product *11f* was isolated by recrystallization from hexane to afford 3.0 g (71.9% yield) of white solid mp 77—80°C. IR (KBr) 1685 (C=0) $cm^{-1}$. NMR ($CDCl_3$) δ 7.50 (d, 1H, J=8Hz, aromatic proton meta to Cl), 6.67 (d, 1H, aromatic proton alpha to Cl), 5.03 (m, 1H, CH), 3.83 (s, 3H, $OCH_3$) 3.03 (m, 2H, $CH_2$) and 1.52 (d, 3H, J=6Hz, $CH_3$).

Anal. Calcd for $C_{11}H_{11}ClO_3$: C, 58.29; H, 4.89. Found: C, 58.27; H, 4.99.

e. Preparation of 4-chloro-2-methyl-2,3-dihydrobenzo[b]furan-7-carboxylic acid (*12f*)

To a solution of 7-carbomethoxy-4-chloro-2-methyl-2,3-dihydrobenzo[b]furan (*11f*) (2.76 g, 12.2 mmol) in 48 ml of methanol there was added 3.0 g (53 mmole) of KOH in 32 ml of water. The solution was stirred at room temperature overnight, and then concentrated under reduced pressure. The residue was treated with 100 ml of water, and then acidified with conc. HCl to pH of about 1. The resulting white solid *12f* was collected on a filter to yield 2.88 g (99.0%) of acid, mp 210—213°C. IR (KBr) 1668 (C=0) $cm^{-1}$. NMR (DMSO-$d_6$) δ 7.63 (d, 1H, J=9Hz, aromatic proton meta to Cl), 6.93 (d, 1H, aromatic proton alpha to Cl), 5.13 (m, 2H, OH, and CH), 3.63—2.60 (m, 2H, $CH_2$) and 1.50 (d, 3H, J=6 Hz, $CH_3$).

f. Preparation of 4-chloro-N-(1-ethyl-2-pyrrolidinylmethyl)-2-methyl-2,3-dihydrobenzo[b]furan-7-carboxamide fumarate (*13f*)

A mixture of 4-chloro-2-methyl-2,3-dihydrobenzo[b]furan-7-carboxylic acid (*12f*) (2.42 g, 11.4 mmol) and thionyl chloride (4.2 ml, 57.0 mmol) in 30 ml of toluene was heated under reflux for 3 hours. The solvent and excess thionyl chloride were removed under reduced pressure to give the corresponding 7-carboxylic acid chloride as a cream colored solid. The acid chloride was then dissolved in 30 ml of $CH_2Cl_2$ and cooled to 0°C in an ice bath. To this there was added dropwise 2-aminomethyl-1-ethylpyrrolidine (1.46 g, 11.4 mmol) in 5 ml of methylene chloride. The solution was stirred at ambient temperature overnight, and then poured into saturated $Na_2CO_3$. The organic layer was separated, dried over anhydrous $Na_2SO_4$ and evaporated to give the desired product as a brown oil (2.77 g). To a solution of the free base in 50 ml of absolute ethanol was added 1.0 g (8.6 mmol) of fumaric acid. The mixture was stirred at room temperature for 2 hours, and cooled in the freezer for 1 hour. The resulting precipitate was collected on a filter and the solid purified by recrystallization from 2-butanone to give *13f* (2.26 g, 45.2% yield) as white crystals, mp 163—164.5°C. IR (nujol) 3350 (NH), 1710 ($CO_2H$), and 1660 (CONH) $cm^{-1}$. NMR (DMSO-$d_6$) δ 8.20 (br s, 1H, NH), 7.63 (d, 1H, J=8Hz, aromatic proton meta to Cl), 7.33 (br s, 2H, COOH), 6.97 (d, 1H, aromatic proton alpha to Cl), 6.57 (S, 2H, 2CH=), 5.17 (m, 1H, CHO), 3.77—1.63 (m, 12H, $6CH_2$), 1.45 (d, 3H, J=6Hz, *$CH_3$* CHO), and 1.13 (t, 3H, J=7Hz, $CH_2$*$CH_3$*).

Anal. Calcd for $C_{17}H_{23}ClN_2O_2.C_4H_4O_4$: C, 57.47; H, 6.20; N, 6.38. Found: C, 57.62; 6.29; N, 6.22.

Synthesis Example 3

a. Preparation of 2,3-dihydrobenzo[b]furan-7-carboxylic acid (*15a*)

To a solution of n-BuLi (1.7 M, 123 ml, 0.21 mol) in 400 ml of hexane at room temperature was added 24.3 g (0.21 mol) of N,N,N',N'-tetramethylethylenediamine (TMEDA), followed by a hexane (40 ml) solution of 2,3-dihydrobenzo[b]furan (*14*) (12.56 g, 0.11 mol). The mixture was stirred under argon at room temperature for 4 hours, and then poured into dry ice (pre-washing with anhydrous ether). After stirring at ambient temperature overnight, the mixture was diluted with water (300 ml), and the layers separated. The aqueous layer was acidified with conc. HCl to pH 1, cooled and the precipitate collected on a filter. This was

recrystallized from $CH_2Cl_2$ to give *15a* (9.43 g, 5.47%) as a white solid, mp 167—169.5°C.

b. Preparation of 5-bromo-2,3-dihydrobenzo[b]furan-7-carboxylic acid (*15b*)

To an ice-cooled acetic acid solution (5 ml) of 2,3-dihydrobenzo[b]furan-7-carboxylic acid (*15a*) (0.33 g. 2.0 mmol) there was added iron (8 mg, 0.14 mmol) and bromine (0.32 g, 2.0 mmol) in 1 ml of acetic acid. The mixture was stirred at room temperature for 18 hours and then poured into water (20 ml). After cooling in the freezer for 1½ hours the product was collected on a filter, and recrystallized from ethyl acetate to give 0.24 g (50.3%) of *15b* as a white crystalline solid, mp 228—229°C.

c. Preparation of 5-nitro-2,3-dihydrobenzo[b]furan-7-carboxylic acid (*15k*)

*Method A:* To an ice-cooled solution of 2,3-dihydrobenzo[b]furan-7-carboxylic acid (*15a*) (0.33 g, 2.0 mmol) in 3 ml of TFA there was added dropwise 0.6 ml of $HNO_3$. At the end of 1 hour, the cooling bath was removed. After an additional 3 hours of stirring, the mixture was poured into ice-water. The precipitate was collected on a filter to give 0.22 g (52.6%) of crude *15k*. This was recrystallized from ethyl acetate to provide 88 mg of acid *15k* (21.4%), mp 249—251.5°C.

*Method B:* To an ice-cooled solution of 2,3-dihydrobenzo[b]furan-7-carboxylic acid (*15a*) (0.33 g, 2.0 mmol) in 3 ml of acetic acid there was added dropwise 0.6 ml of $HNO_3$. The mixture was stirred in a warm oil bath (70°C) for 24 hours, and then poured into water. After cooling, the product was collected on a filter to give 0.21 g of solid (50.2%). This was recrystallized from ethyl acetate to afford 0.14 g (33.5%) of *15k*, mp 249—251.5°C.

d. Preparation of N-(1-ethyl-2-pyrrolidinylmethyl)-5-nitro-2,3-dihydrobenzo[b]furan-7-carboxamide (*16k*)

A mixture of (*15k*) (3.0 mmol) and thionyl chloride (12.3 mmol) in 15 ml of toluene was heated under reflux for 3 hours. The solvent and excess of thionyl chloride was removed under reduced pressure to give the corresponding 7-carboxylic acid chloride. This was then dissolved in 15 ml methylene chloride and cooled to 0°C in an ice bath. To this there was added dropwise 2-aminomethyl-1-ethylpyrrolidine (3.0 mmol) in 5 ml of methylene chloride. The yellow solution was stirred at ambient temperature overnight, and then taken to dryness in vacuum. The residue was recrystallized from 2-butanone to give *16k* as a solid.

Synthesis Example 4

a. Preparation of 2-(2,6-dibromo-4-methylphenyloxy)ethyl bromide (*19*)

A mixture of 2,6-dibromo-4-methylphenyl) (*18*) (26.59 g, 0.1 mol), 1,2-dibromoethane (11.2 ml, 0.13 mol) and potassium hydroxide (8.42 g, 0.15 mol) in 200 ml of absolute ethanol was heated under reflux for 24 hours. The inorganic salt was collected on a filter and the filtrate taken to dryness. The resulting mixture was treated with ethyl ether, chilled, and the solid, which proved to be recovered phenol, collected on a filter. The filtrate was evaporated to afford the ether *19* (24.5 g, 65.7%) as a liquid. This was purified by distillation (0.5 torr, 120—124°C) to yield 19.79 g (53.0%) of product. NMR ($CDCl_3$) δ 7.25 (s, 2H, aromatic protons), 3.55—4.38 (m, 4H, $2CH_2$), and 2.27 (s, 3H, $CH_3$).

b. Preparation of 5-methyl-2,3-dihydrobenzo[b]furan-7-carboxylic acid (*20*)

To a stirred solution of 2-(2,6-dibromo-4-methylphenyloxy)ethyl bromide (*19*) (5.82 g, 15.6 mmol) in dry THF (90 ml) and hexane (30 ml) under argon there was added 7.8 ml of n-BuLi (2.5 M in hexane) at 78°C (dry ice/acetone bath). After 30 minutes at 78°C, an additional 7.8 ml of n-BuLi was added. After an additional 1 hour stirring, the light yellow reaction solution was poured into a slurry of dry ice (prewashed with dry ether). After the mixture had come to room temperature, the precipitated white solid was collected on a filter, treated with 1N HCl to adjust the pH of the supernatant liquid to 1. The mixture was cooled briefly in the freezer, and the solid collected on a filter to afford 2.52 g (90.7%) of acid *20*. The crude product was recrystallized from $CH_3OH/H_2O$ to yield 1.70 g (61.2%) of *3* as a white solid, mp 177—179°C. IR(KBr) 3420 (OH) and 1680(C=O) $cm^{-1}$. NMR($CDCl_3$) δ 8.47 (br s, 1H, OH), 7.50 (s, 1H, aromatic proton), 7.15 (s, 1H, aromatic proton), 4.70 (t, 2H, J=9Hz, $OCH_2$), 3.20 (t, 2H, $OCH_2CH_2$), and 2.32 (s, 3H, $CH_3$).

Anal. Calcd for $C_{10}H_{10}O_3$: C, 67.41; H, 5.66. Found: C, 67.45; H, 5.78.

c. Preparation of N-(1-ethyl-2-pyrrolidinylmethyl)-5-methyl-2,3-dihydrobenzo[b]furan-7-carboxamide hydrochloride (*21*)

A mixture of 2-methyl-2,3-dihydrobenzo[b]furan-7-carboxylic acid (*20*) (3.43 g, 19.25 mmol) and thionyl chloride (7.0 ml, 96.2 mmol) in 40 ml of toluene was heated under reflux for 3 hours. The solvent and excess of thionyl chloride were removed under reduced pressure to give the corresponding 7-carboxylic acid chloride. This was then dissolved in 25 ml of $CH_2Cl_2$ and cooled at 0°C with an ice bath. To this there was added dropwise 2-aminomethyl-1-ethylpyrrolidine (2.47 g, 19.25 mmol) in 10 ml of $CH_2Cl_2$. The solution was stirred overnight at room temperature and then evaporated to dryness to afford crude carboxamide (6.24 g, 100%). This was purified by recrystallization from acetone to give 5.29 g (84.6%) as white crystals, mp 203.5—205°C. IR (nujol) 3340 (NH), and 1645 (C=O) $cm^{-1}$. NMR (DMSO δ 8.13 (br s, 1H, NH), 7.25 (s, 1H, aromatic proton), 7.10 (s, 1H, aromatic proton), 4.58 (t, 2H, J=9 Hz, $OCH_2$), 1.88 (s, 3H, $CH_3$), and 1.25 (t, 3H, J=7Hz, $CH_2CH_3$).

Anal. Calcd for $C_{17}H_{24}N_2O_2.HCl$: C, 62.85; H, 7.76; N, 8.62. Found: C, 62.99; H, 7.79; N, 8.56.

Synthesis Example 5

a. Preparation of 5-amino-N-(1-ethyl-2-pyrrolidinylmethyl)-2,3-dihydrobenzo[b]furan-7-carboxyamide fumarate (*16l*)

A mixture of N - (1 - ethyl - 2 - pyrrolidinylmethyl) - 5 - nitro - 2,3 - dihydrobenzo[b]furan - 7 - carboxamide (*16k*) (1.08 g, 3.4 mmol) and palladium on carbon (5%) (0.10 g) in 25 ml of absolute ethanol was shaken under hydrogen for 18 hours. The catalyst was collected on a filter with the aid of Celite and the filtrate evaporated. The residue was purified by column chromatography (silica gel, $NH_4OH/CH_3OH/CH_2Cl_2$ = 0.1/1/100 to 0.1 to 0.1/6/100). The product was isolated as the fumarate and recrystallized from 2-butanone to afford 0.57 g (41.4%) of *16l* as a yellow solid, mp 98—101°C.

Synthesis Example 6

a. Preparation of 5-acetamido-N-(1-ethyl-2-pyrrolidinylmethyl)-2,3-dihydrobenzo[b]furan-7-carboxamide fumarate (*16m*)

A solution of 5 - amino - N - (1 - ethyl - 2 - pyrrolidinylmethyl) - 2,3 - dihydrobenzo[b]furan - 7 - carboxamide (*16l*) (1.02 g, 3.5 mmol) in 10 ml of acetic anhydride was stirred at room temperature overnight. The mixture was added to 50 ml of ethyl ether, cooled and the precipitate collected on a filter. This solid was dissolved in 50 ml of $CH_2Cl_2$, and washed with saturated $NaHCO_3$ (2 × 50 ml). The organic layer was dried over anhydrous $Na_2SO_4$, and evaporated to give the free base (1.03 g, 88.8%). The product was characterized as its fumarate; there was obtained 1.26 g of the salt (80.3%), mp 179—181°C (EtOH).

Synthesis Example 7

a. Preparation of N-(1-ethyl-2-pyrrolidinylmethyl)-5-methanesulfonylamido-2,3-dihydrobenzo[b]furan-7-carboxamide (*16n*)

A solution of 5 - amino - N - (1 - ethyl - 2 - pyrrolidinylmethyl) - 2,3 - dihydrobenzo[b]furan - 7 - carboxamide (*16l*) (1.80 g. 6.2 mmol) and methanesulfonyl chloride (12.78 g. 0.11 mol) in 30 ml of methylene chloride was heated to reflux overnight. The solution was diluted with $CH_2Cl_2$ (30 ml), and then extracted with water (2 × 30 ml). The combined aqueous layers were made basic with $NaHCO_3$ powder, and extracted with $CH_2Cl_2$ (4 × 50 ml). The organic extracts were combined, dried over anhydrous $Na_2SO_4$, and evaporated to give *16n* (2.25 g, 98.8%). This was recrystallized from EtOH, charcoal to afford 1.70 g of amide as a white solid, mp 180—182°C.

Hereinabove the invention has been described by reference to the synthesis of pyrrolidinylmethyl derivatives by reacting benzo[b]furan-7-carboxylic or dihydrobenzo[b]furan-7-carboxylic acid or the corresponding esters or acid chlorides with 2-aminomethyl-1-ethylpyrroline. Other benzofuran carboxamides as defined in A in general formula I can be prepared in an analogous manner by reacting the aforementioned acids, esters or acid chlorides with other amines as shown in Synthesis Example 8 to 10 below.

Synthesis Example 8

a. Preparation of 5-bromobenzo[b]furan-7-carboxylic acid chloride

A mixture of 5-bromobenzo[b]furan-7-carboxylic acid (5.5 g, 22.8 mmol) and thionyl chloride (6.7 ml, 91.9 mmol) in 40 ml of toluene was heated under reflux for 3 hours. The solvent and excess thionyl chloride were then removed under reduced pressure to give *8b* (5.04 g, 85.2%) as a yellow solid. This was used for subsequent reactions with amines without purification.

b. Preparation of 5-bromo-N-(2-diethylaminoethyl)benzo[b]furan-7-carboxamide hydrochloride

To an ice-cooled solution of 5-bromobenzo[b]furancarboxylic acid chloride (1.5 g, 5.8 mmol) in 15 ml of $CH_2Cl_2$, there was added N,N-diethylethylenediamine (0.74 g, 6.4 mmol) in 10 ml of $CH_2Cl_2$. After stirring at ambient temperature overnight, the precipitate was collected on a filter to give the crude amide (1.14 g, 53.2%). This was recrystallized from ethanol (decolorization with charcoal) to give 0.54 g (24.8%) of the desired amide as a white solid, mp 202—203.5°C. IR (KBr) 1675 (C=0) $cm^{-1}$.

Anal. Calcd for $C_{15}H_{19}BrN_2O_2$.HCl: C, 47.95; H, 5.37; N, 7.46. Found: C, 47.87; H, 5.48; N, 7.33.

Synthesis Example 9

a. Preparation of [exo]-5-bromo-N-[8-(phenylmethyl)-8-azabicyclo[3,2,1]oct-3-yl]benzo[b]furan-7-carboxamide

To an ice-cooled solution of 5-bromobenzo[b]furan-7-carboxylic acid chloride prepared as in Synthesis Example 8 (1.5 g, 5.8 mmol) in 15 ml of $CH_2Cl_2$, there was added 8-benzyl-3β-amino-1αH,5αH-nortropane (1.25 g, 5.8 mmol) in 10 ml of $CH_2Cl_2$. The mixture was stirred at ambient temperature overnight and then poured into saturated aqueous $NaHCO_3$. The layers were separated, and the organic layer dried over anhydrous $Na_2SO_4$. Evaporation of solvent gave crude amide (2.28 g, 89.5%). The analytical sample was obtained by two recrystallizations from ethanol to give a sample of the target amide pure (0.81 g, 31.8%) as a white crystalline solid, mp 161—163°C. IR (KBr) 1662 (C=0) $cm^{-1}$.

Anal. Calcd for $C_{23}H_{23}BrN_2O_2$: C, 62.88; H, 5.28; N, 6.38. Found: C, 62.99; H, 5.37; N, 6.27.

Synthesis Example 10

a. Preparation of N-(1-benzyl-3-pyrrolidinyl)-5-bromobenzo[b]furan-7-carboxamide fumarate

To an ice-cooled solution of 5-bromobenzo[b]furan-7-carboxylic acid chloride prepared as in Synthesis Example 8 (1.82 g, 7.0 mmol) in 25 ml of $CH_2Cl_2$, there was added 3-amino-1-benzylpyrrolidine (1.24 g, 7.0 mmol)in 10 ml of $CH_2Cl_2$. The mixture was stirred at ambient temperature overnight, and then poured into saturated aqueous $NaHCO_3$. The layers were separated, and the organic layer dried over anhydrous $Na_2SO_4$. Evaporation of the solvent afforded the desired product as its free base (1.93 g, 68.9%). This was more conveniently isolated as its fumarate by adding to the free base in 15 ml of absolute ethanol 0.56 g of fumaric acid, and the mixture stirred at room temperature for 1 hour. The resulting precipitate was collected on a filter to give the salt (2.12 g, 58.6%). This was recrystallized from ethanol to furnish an analytically pure sample of the desired amide (1.10 g, 30.5%) as a beige solid, mp 197—200°C. IR (KBr) 1710 (COOH), 1655 (CONH) $cm^{-1}$.

Anal. Calcd. for $C_{20}H_{19}BrN_2O_2.C_4H_4O_4$: C, 55.93; H, 4.50; N, 5.44. Found: C, 56.44; H, 4.85; N, 5.81.

In Vivo Screening Tests

a. Antipsychotic Screen

The effect of each of the benzofurancarboxamides shown in Table VIII below on apomorphine induced climbing behavior was tested under the conditions described by Costetin et al, "Rapid and Dissociated Changes in Sensitivities of Different Dopamine Receptors in Mouse Brain", *Nature*, 257, 405 (1975). Each mouse was administered from 0.1 to 50 mg/kg of test compound and antagonism was observed 20 minutes after injection of 1 mg, kg apomorphine induced climbing behaviour. The ED 50 values are shown in Table VIII.

b) Antiemetic Screen

Each of the test compounds shown in Table VIII was administered subcutaneously to two beagle dogs at dosage levels of 0.05, 0.1, 1.0 and 10.0 mg,kg. One hour following administration of the test compound, each animal received 0.1 mg, kg, *sc*, of apomorphine HCl. Apomorphine consistently evokes an immediate emetic response through direct dopaminergic interaction at the medullary chemoreceptor trigger zone (CTZ). Antiemetic compounds which act at the CTZ and, or the midbrain vomiting center will block this effect. If both animals receiving a particular treatment are protected, (i.e., no emetic response within one hour after apomorphine), the test compound is considered active. The dosage at which both animals were protected (100% effect) is shown in Table VIII.

TABLE VIII

Effects of Benzofuran/Dihydrobenzofuran Compounds *In Vivo* Screening Tests

| Structure | Adomorphine Emesis (Dog) 100% Effect Dosage level mg/kg, s.c. | Apomorphine Climbing (Mouse) Approx. 50% ED (mg/kg, s.c.) |
|---|---|---|
| 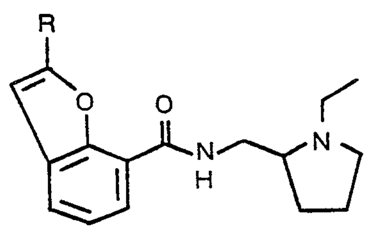 | | |
| R=H | 10.0 | 12 (i.p.) |
| R=$CH_3$ | 10.0 | 3 (i.p.) |
| 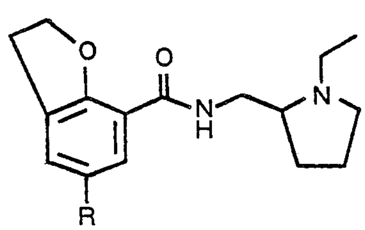 | | |
| R=H | 1.0 | 20 (i.p.) |
| R=Cl | 1.0 | 15 (oral)<br>5 (i.p.) |

TABLE VIII CONTINUED
Effects of Benzofuran/Dihydrobenzofuran Compounds *In Vivo* Screening Tests

| Structure | Adomorphine Emesis (Dog) 100% Effect Dosage level mg/kg, s.c. | Apomorphine Climbing (Mouse) Approx. 50% ED (mg/kg, s.c.) |
|---|---|---|
| R=$NO_2$ | 1.0 | 20 (i.p.)<br>15 (oral) |
| R=Br | 1.0 | 15 (oral) |
| R=$NH_2$ | — | 25 (i.p.)<br>20 (oral) |
| R=$NHCOCH_3$ | — | 50 (i.p.) |
| R=$NHSO_2CH_3$ | 1.0 | 50 (oral) |
| R=$CH_3$ | 0.1 | 3 (i.p.) |
| 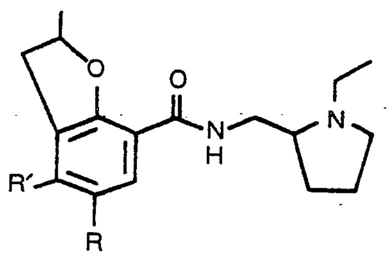 | | |
| R=H R′=H | 0.05 | 20 (i.p.) |
| R=Br R′=H | 0.005 | 2 (i.p.) |
| R=$OCH_3$ R′=H | 0.1 | 25 (i.p.)<br>1 (oral) |
| R=Cl R′=Cl | — | 10 (oral) |
| R=H R′=$OCH_3$ | — | 25 (oral) |
| R=$NH_2$ R′=Cl | — | 25 (i.p.)<br>50 (oral) |
| R=H R′=Cl | — | 25 (i.p.)<br>40 (oral) |
| 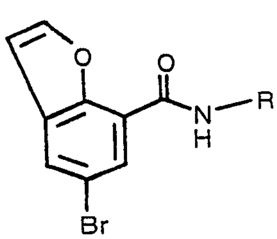 | | |
| R= 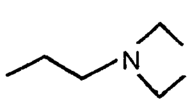 | 1.0 | 25. (i.p.)<br>5 (oral) |

TABLE VIII CONTINUED
Effects of Benzofuran/Dihydrobenzofuran Compounds *In Vivo* Screening Tests

| Structure | Adomorphine Emesis (Dog) 100% Effect Dosage level mg/kg, s.c. | Apomorphine Climbing (Mouse) Approx. 50% ED (mg/kg, s.c.) |
|---|---|---|
| R= 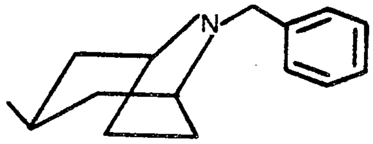 | — | 10 (i.p.)<br>10 (oral) |
| R= 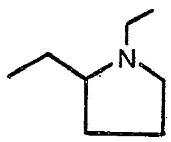 | 0.1 | 0.5 (i.p.) |
| R= 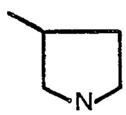 | — | 30 (i.p.) |
| 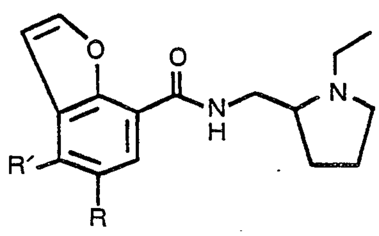 | | |
| R=H R′=H | 10.0 | 12 (i.p.) |
| R=Cl R′=H | 0.01 | 5 (oral) |
| R=Br R′=H | 0.1 | — |
| R=F R′=H | 1.0 | — |
| R=H R′=Cl | — | — |
| 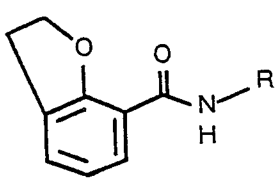 | | |
| R= 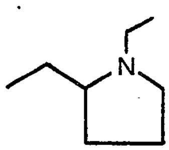 | 1.0 | 20 (i.p.) |
| R= 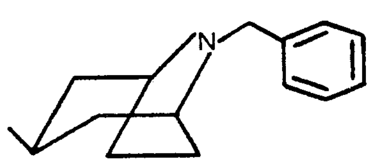 | 1.0 | 20 (i.p.)<br>15 (oral) |

TABLE VIII CONTINUED
Effects of Benzofuran/Dihydrobenzofuran Compounds *In Vivo* Screening Tests

| Structure | Adomorphine Emesis (Dog) 100% Effect Dosage level mg/kg, s.c. | Apomorphine Climbing (Mouse) Approx. 50% ED (mg/kg, s.c.) |
|---|---|---|
| 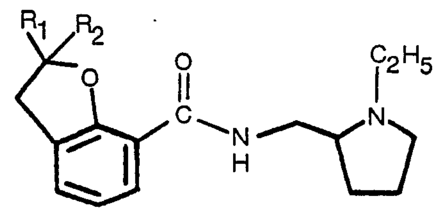 | | |
| $R_1=R_2=H$ | 1.0 | 20 (i.p.) |
| $R_1=H, R_2=CH_3$ | 0.85 | 20 (i.p.) |
| $R_1=R_2=CH_3$ | 10.0 | 100 (i.p.) |

**Claims for the Contracting States: BE IT LU SE**

1. A compound of the formula (I)

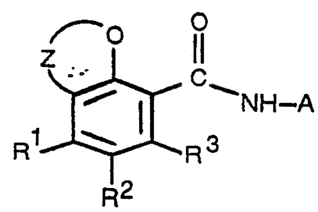

I

wherein Z represents the atoms necessary to complete a benzo[b]furan ring dihydrobenzo[b]furan ring, either of which may be substituted with an alkyl group, $R^1$, $R^2$ and $R^3$ may be the same or different and each represent a hydrogen atom, an alkyl group having 1 to 6 carbon atoms, a halogen atom, an amino group, an alkylamino group in which the alkyl substituent(s) contain 1 to 6 carbon atoms, an alkoxy group having 1 to 6 carbon atoms, an acylamido group having 1 to 6 carbon atoms, a sulfonamido group having up to 6 carbon atoms and being attached to the benzo[b]furan system by the N-atom, or a nitro group; and A represents an aminoalkyl group containing the atomic sequence —C—C—N, and acid addition salts thereof.

2. A compound of the formula II

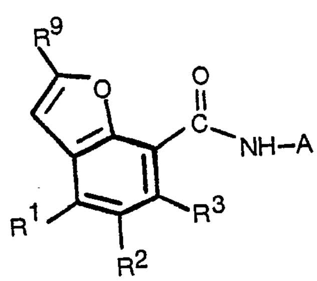

II

wherein A, $R^1$, $R^2$ and $R^3$ are as defined in claim 1, and $R^9$ represents a hydrogen atom or an alkyl group containing 1 to 3 carbon atoms; and acid addition salts thereof.

3. A compound of the formula III

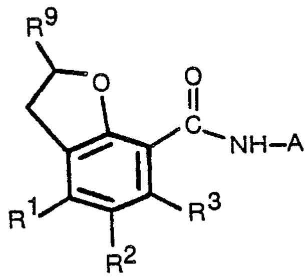

III

wherein A, $R^1$, $R^2$ and $R^3$ are as defined in claim 1, and $R^9$ represents a hydrogen atom or an alkyl group containing 1 to 3 carbon atoms; and acid addition salts thereof.

4. A compound of the formula (IV):

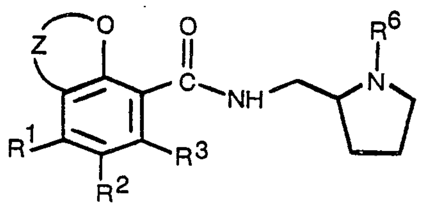

IV

wherein Z represents the atoms necessary to complete an unsubstituted or alkyl-substituted benzo[b]furan or dihydrobenzo[b]furan ring, and $R^1$, $R^2$ and $R^3$ are as defined in claim 1, and $R^6$ represents a hydrogen atom or an alkyl group having 1 to 6 carbon atoms; and acid addition salts thereof.

5. A compound according to any one of claims 1 to 3, wherein A represents a substituent selected from:

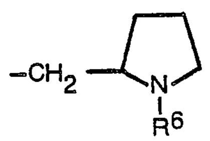

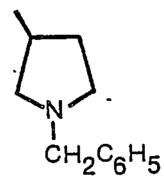

$-CH_2CH_2NR^7R^8$

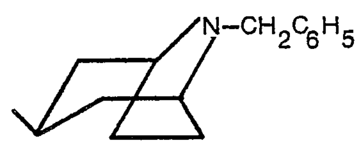

wherein $R^6$, $R^7$ and $R^8$ each represent a hydrogen atom or an alkyl group containing 1 to 6 carbon atoms.

6. A compound according to claim 5 wherein A represents a 2-pyrrolidinylmethyl group.

7. A compound according to claim 1 or claim 4 wherein Z represents the atoms necessary to complete a 2-methyl or 3-methyl benzo[b]furan ring.

8. A compound according to claim 1 or claim 4 wherein Z represents the atoms necessary to complete a 2-methyl or 3-methyl 2,3-dihydrobenzo[b]furan ring.

9. A compound according to any preceding claim wherein at least one of $R^1$, $R^2$ and $R^3$ is a halogen atom.

10. A compound according to any preceding claim wherein at least one of $R^1$, $R^2$ and $R^3$ is a methoxy group.

11. A compound according to any preceding claim wherein at least one of $R^1$, $R^2$ and $R^3$ is an acylamido group.

12. A compound according to any preceding claim wherein at least one of $R^1$, $R^2$ and $R^3$ is a sulfonamido group as defined in claim 1.

13. A compound according to any preceding claim wherein at least one of $R^1$, $R^2$ and $R^3$ is an amino group or an alkylamino group.

14. A compound according to any preceding claim wherein at least one of $R^1$, $R^2$ and $R^3$ is a nitro group.

15. A compound according to any preceding claim wherein two of the substituents $R^1$, $R^2$ and $R^3$ represent hydrogen atoms.

16. A compound according to claim 15 wherein two of $R^1$, $R^2$ and $R^3$ represent a hydrogen atom and the other is a methoxy group, an acylamido group or a sulfonamido group as defined in claim 1.

17. A compound according to claim 4 wherein $R^6$ represents a hydrogen atom or an alkyl group having one to six carbon atoms.

18. A compound according to any preceding claim for use as an antipsychotic or antiemetic agent.

19. A pharmaceutical preparation useful as an antipsychotic or antiemetic agent comprising a pharmaceutically acceptable carrier in combination with a therapeutically effective amount of a compound according to any preceding claim.

20. A process for producing a benzofurancarboxamide having the general formula:

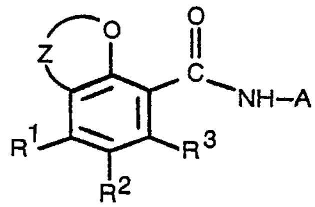

I

wherein Z represents the atoms necessary to complete a benzo b furan ring or a dihydrobenzo b furan ring, either of which may be substituted with an alkyl group, $R^1$, $R^2$ and $R^3$ may be the same or different and each represent a hydrogen atom, an alkyl group having 1 to 6 carbon atoms, a halogen atom, an amino group, an alkylamino group in which the alkyl substituent(s) contain 1 to 6 carbon atoms, an alkoxy group having 1 to 6 carbon atoms, an acylamido group having 1 to 6 carbon atoms, a sulfonamido group having up to 6 carbon atoms and being attached to the benzo[b]furan system by the N-atom, or a nitro group; and A represents a tertiary aminoalkyl group containing the atomic sequence

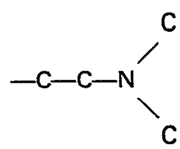 ,

wherein the nitrogen atom may form part of a pyrrolidinyl ring or an acid addition salt thereof, which comprises condensing a benzo- or dihydrobenzo-[b]furan-7-carboxylic acid chloride or ester with an amine and recovering a carboxamide in the form of its acid salt.

**Claims for the Contracting States: CH DE FR GB NL**

1. A compound of the formula (I)

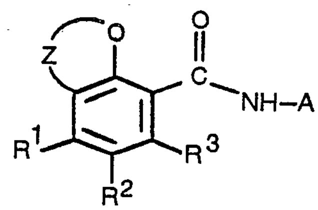

I

wherein Z represents the atoms necessary to complete a benzo[b]furan ring dihydrobenzo[b]furan ring, either of which may be substituted with an alkyl group $R^1$, $R^2$ and $R^3$ may be the same or different and each represent a hydrogen atom, an alkyl group having 1 to 6 carbon atoms, a halogen atom, an amino group, an alkylamino group in which the alkyl substituent(s) contain 1 to 6 carbon atoms, an alkoxy group having 1 to 6 carbon atoms, an acylamido group having 1 to 6 carbon atoms, a sulfonamido group having up to 6 carbon atoms and being attached to the benzo[b]furan system by the N-atom, or a nitro group; and A represents a tertiary amino alkyl group which contains the atomic sequence —C—C—N and wherein the nitrogen atom may form part of a pyrrolidinyl ring, and acid addition salts thereof.

2. A compound of the formula II

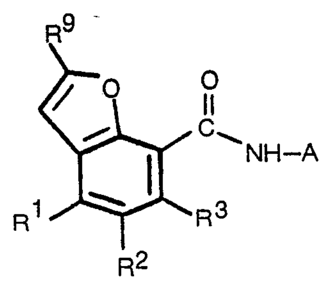

II

wherein A, $R^1$, $R^2$ and $R^3$ are as defined in claim 1, and $R^9$ represents a hydrogen atom or an alkyl group containing 1 to 3 carbon atoms; and acid addition salts thereof.

3. A compound of the formula III

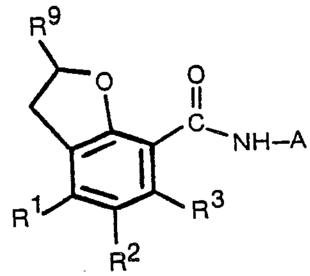

III

wherein A, $R^1$, $R^2$ and $R^3$ are as defined in claim 1, and $R^9$ represents a hydrogen atom or an alkyl group containing 1 to 3 carbon atoms; and acid addition salts thereof.

4. A compound of the formula (IV):

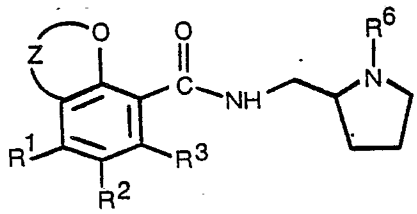

IV

wherein Z represents the atoms necessary to complete an unsubstituted or alkyl-substituted benzo[b]furan or dihydrobenzo[b]furan ring, and $R^1$, $R^2$ and $R^3$ are as defined in claim 1, and $R^6$ represents a hydrogen atom or an alkyl group having 1 to 6 carbon atoms; and acid addition salts thereof.

5. A compound according to any one of claims 1 to 3, wherein A represents a substituent selected from:

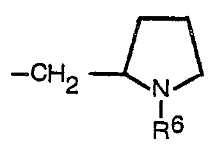

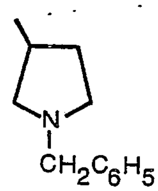

$-CH_2CH_2NR^7R^8$

wherein $R^6$, $R^7$ and $R^8$ each represent a hydrogen atom or an alkyl group containing 1 to 6 carbon atoms.

6. A compound according to claim 5 wherein A represents a 2-pyrrolidinylmethyl group.

7. A compound according to claim 1 or claim 4 wherein Z represents the atoms necessary to complete a 2-methyl or 3-methyl benzo[b]furan ring.

8. A compound according to claim 1 or claim 4 wherein Z represents the atoms necessary to complete a 2-methyl or 3-methyl 2,3-dihydrobenzo[b]furan ring.

9. A compound according to any preceding claim wherein at least one of $R^1$, $R^2$ and $R^3$ is a halogen atom.

10. A compound according to any preceding claim wherein at least one of $R^1$, $R^2$ and $R^3$ is a methoxy group.

11. A compound according to any preceding claim wherein at least one of $R^1$, $R^2$ and $R^3$ is a acylamido group.

12. A compound according to any preceding claim wherein at least one of $R^1$, $R^2$ and $R^3$ is a sulfonamido group as defined in claim 1.

13. A compound according to any preceding claim wherein at least one of $R^1$, $R^2$ and $R^3$ is an amino group or an alkylamino group.

14. A compound according to any preceding claim wherein at least one of $R^1$, $R^2$ and $R^3$ is a nitro group.

15. A compound according to any preceding claim wherein two of the substituents $R^1$, $R^2$ and $R^3$ represent hydrogen atoms.

16. A compound according to claim 15 wherein two of $R^1$, $R^2$ and $R^3$ represent a hydrogen atom and the other is a methoxy group, an acylamido group or a sulfonamido group as defined in claim 1.

17. A compound according to claim 4 wherein $R^6$ represents a hydrogen atom or an alkyl group having one to six carbon atoms.

18. A compound according to any preceding claim for use as an antipsychotic or antiemetic agent.

19. A pharmaceutical preparation useful as an antipsychotic or antiemetic agent comprising a pharmaceutically acceptable carrier in combination with a therapeutically effective amount of a compound according to any preceding claim.

20. A process for producing a benzofurancarboxamide having the general formula:

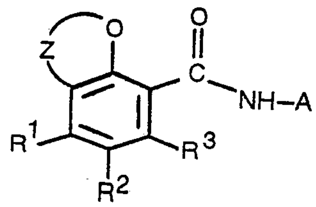

I

wherein Z represents the atoms necessary to complete a benzo b furan ring or a dihydrobenzo b furan ring, either of which may be substituted with an alkyl group, $R^1$, $R^2$ and $R^3$ may be the same or different and each represent a hydrogen atom, an alkyl group having 1 to 6 carbon atoms, a halogen atom, an amino group, an alkylamino group in which the alkyl substituent(s) contain 1 to 6 carbon atoms, an alkoxy group having 1 to 6 carbon atoms, an acylamido group having 1 to 6 carbon atoms, a sulfonamido group having up to 6 carbon atoms and being attached to the benzo[b]furan system by the N-atom, or a nitro group; and A represents a tertiary aminoalkyl group containing the atomic sequence —C—C—N, or an acid addition salt thereof, which comprises condensing a benzo- or dihydrobenzo-[b]furan-7-carboxylic acid, acid chloride or ester having the general formula:

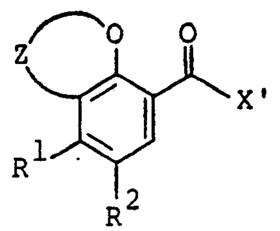

wherein $R^1$, $R^2$ and Z are as defined above and $X^1$ represents a chlorine atom or a hydroxyl or alkoxy group, with an amine having the general formula A—$NH_2$, wherein A is as defined above.

**Claims for the Contracting State: AT**

1. A process for producing a benzofurancarboxamide having the general formula:

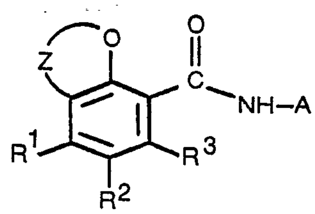

I

wherein Z represents the atoms necessary to complete a benzo[b]furan ring dihydrobenzo[b]furan ring, either of which may be substituted with an alkyl group, $R^1$, $R^2$ and $R^3$ may be the same or different and each represent a hydrogen atom, an alkyl group having 1 to 6 carbon atoms, a halogen atom, an amino group, an alkylamino group in which the alkyl substituent(s) contain 1 to 6 carbon atoms, an alkoxy group having 1 to 6 carbon atoms, an acylamido group having 1 to 6 carbon atoms, a sulfonamido group having up to 6 carbon atoms and being attached to the benzo[b]furan system by the N-atom, or a nitro group; and A represents an aminoalkyl group containing the atomic sequence —C—C—N, or an acid addition salt thereof, which comprises condensing a benzo- or dihydrobenzo-[b]furan-7-carboxylic acid, acid chloride or ester having the general formula

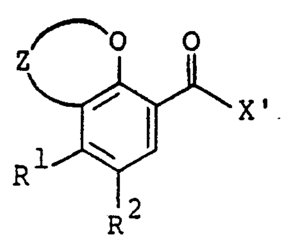

wherein $R^1$, $R^2$ and Z are as defined above and $X^1$ represents a chlorine atom or a hydroxyl or alkoxy group, with an amine having the general formula A—$NH_2$, wherein A is as defined above.

2. A process according to claim 1 wherein the said carboxylic acid, acid chloride or ester is a compound of the formula:

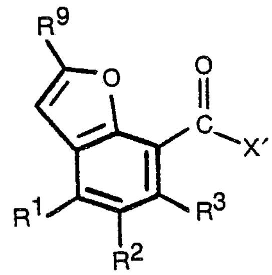

wherein $X^1$, $R^1$, $R^2$ and $R^3$ are as defined in claim 1, and $R^9$ represents a hydrogen atom or an alkyl group containing 1 to 3 carbon atoms.

3. A process according to claim 1 wherein the said carboxylic acid, acid chloride or ester is a compound of the formula:

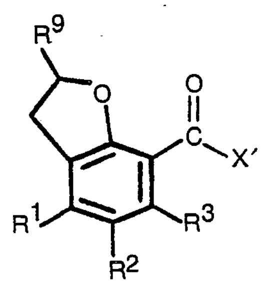

wherein $X^1$, $R^1$, $R^2$ and $R^3$ are as defined in claim 1, and $R^9$ represents a hydrogen atom or an alkyl group containing 1 to 3 carbon atoms.

4. A process according to any preceding claim wherein the amine has one of the following formulas:

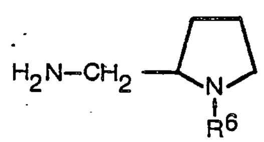

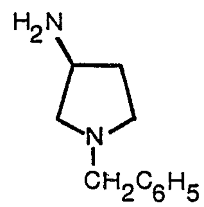

$H_2N-CH_2CH_2NR^7R^8$

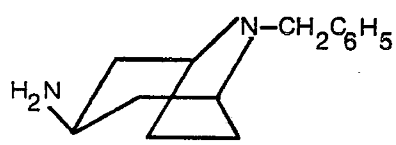

wherein $R^6$, $R^7$ and $R^8$ each represent a hydrogen atom or an alkyl group containing 1 to 6 carbon atoms.

5. A process according to claim 4 wherein A represents a 2-pyrrolidinylmethyl group.

6. A process according to claim 1 or claim 4 wherein Z represents the atoms necessary to complete a 2-methyl or 3-methyl benzo[b]furan ring.

7. A process according to claim 1 or claim 4 wherein Z represents the atoms necessary to complete a 2-methyl or 3-methyl 2,3-dihydrobenzo[b]furan ring.

8. A process according to any preceding claim wherein at least one of $R^1$, $R^2$ and $R^3$ is a methoxy group.

9. A process according to any preceding claim wherein the said carboxylic acid ester is a methyl ester and is obtained by:

Alkylating the 2-hydroxyl group of substituted or unsubstituted methyl salicylate, having groups $R^1$, $R^2$ at the 4- and 5-positions respectively, by a nucleophilic substitution with an alkyl halide;

subjecting the intermediate thus obtained to a claisen rearrangement to give a methyl (3-alkyl salicylate);

oxidising the alkyl substituent to an aldehyde or ketone group and

reacting the 2-hydroxy group with the aldehyde or ketone group to effect a cyclization.

10. A process according to claim 9 wherein the cyclization is carried out in the presence of an acid catalyst to produce a benzo[b]furan ring.

11. A process according to claim 9 wherein the cyclization is carried out by treating the methyl (3-alkyl salicylate) with mercuric acetate followed by sodium borohydride to give a dihydrobenzo-[b]-furan ring.

12. A process according to claim 3 wherein the amine is reacted with a 2,3-dihydrobenzo[b]furan-7-carboxylic acid produced by lithiation of 2,3-dihydrobenzo[b]furan with n-butyl lithium, followed by quenching with dry ice and acidification with concentrated H Cl.

13. A process according to claim 2 wherein the amine is reacted with a benzo[b]furan-7-carboxylic acid produced by alkylating a 2,6-dibromophenol with 1,2-dibromoethane to produce a phenyloxyethyl bromide which is then lithiated with n-butyl lithium quenched in dry ice and acidified.

**Patentansprüche für die Vertragsstaaten: BE IT LU SE**

1. Verbindung der Formel (I)

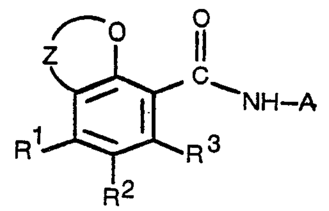

I

worin Z die erforderlichen Atome bedeutet, um einen Benzo[b]furanring oder einen Dihydrobenzo[b]-furanring zu vervollständigen, von denen jeder mit einer Alkylgruppe substituiert sein kann, $R^1$, $R^2$ und $R^3$ gleich oder verschieden sein können und jeweils ein Wasserstoffatom, eine Alkylgruppe mit 1 bis 6 Kohlenstoffatomen, ein Halogenatom, eine Aminogruppe, eine Alkylaminogruppe, in welcher der oder die Alkylsubstituenten 1 bis 6 Kohlenstoffatome enthalten, eine Alkoxygruppe mit 1 bis 6 Kohlenstoffatomen, eine Acylamidògruppe mit 1 bis 6 Kohlenstoffatomen, eine Sulfonamidogruppe mit bis zu 6 Kohlenstoffatomen, die an das Benzo[b]furansystem durch das N-Atom gebunden ist, oder eine Nitrogruppe bedeutet und A eine Aminoalkylgruppe ist, die die Atomfolge —C—C—N enthält, und deren Säureadditionssalze.

2. Verbindung der Formel II

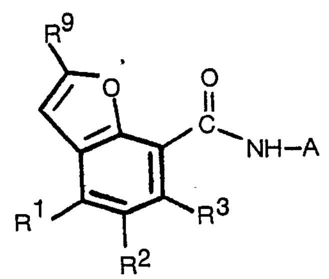

II

worin A, $R^1$, $R^2$ und $R^3$ wie in Anspruch 1 definiert sind und $R^9$ ein Wasserstoffatom oder eine Alkylgruppe, die 1 bis 3 Kohlenstoffatome enthält, bedeutet, und deren Säureadditionssalze.

3. Verbindung der Formel III

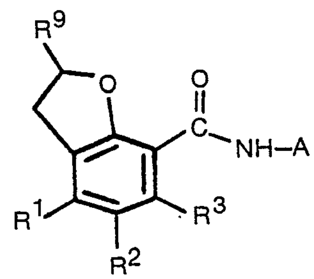

III

worin A, $R^1$, $R^2$ und $R^3$ wie in Anspruch 1 definiert sind und $R^9$ ein Wasserstoffatom oder eine Alkylgruppe, die 1 bis 3 Kohlenstoffatome enthält, bedeutet, und deren Säureadditionssalze.

4. Verbindung der Formel IV

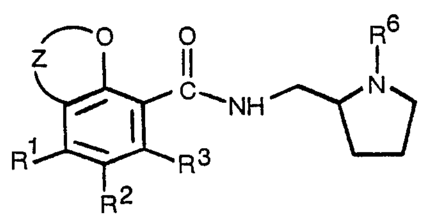

IV

worin Z die erforderlichen Atome bedeutet, um einen unsubstituierten oder alkylsubstituierten Benzo[b]-furan- oder Dihydrobenzo[b]furanring zu vervollständigen, und $R^1$, $R^2$ und $R^3$ wie in Anspruch 1 definiert sind und $R^6$ ein Wasserstoffatom oder eine Alkylgruppe mit 1 bis 6 Kohlenstoffatomen bedeutet, und deren Säureadditionssalze.

5. Verbindung nach einem der Ansprüche 1 bis 3, worin A einen der Substituenten

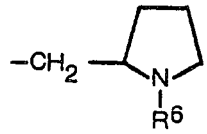

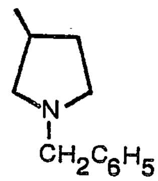

$-CH_2CH_2NR^7R^8$

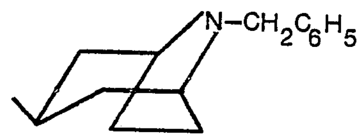

bedeutet, worin $R^6$, $R^7$ und $R^8$ jeweils ein Wasserstoffatom oder eine Alkylgruppe mit 1 bis 6 Kohlenstoffatomen bedeutet.

6. Verbindung nach Anspruch 5, worin A eine 2-Pyrrolidinylmethylgruppe bedeutet.

7. Verbindung nach Anspruch 1 oder Anspruch 4, worin Z die erforderlichen Atome bedeutet, um einen 2-Methyl- oder 3-Methylbenzo[b]furanring zu vervollständigen.

8. Verbindung nach Anspruch 1 oder Anspruch 4, worin Z die erforderlichen Atome bedeutet, um einen 2-Methyl- oder 3-Methyl-2,3-dihydrobenzo[b]furanring zu vervollständigen.

9. Verbindung nach einem der vorausgehenden Ansprüche, worin wenigstens einer der Substituenten $R^1$, $R^2$ und $R^3$ ein Halogenatom ist.

10. Verbindung nach einem der vorausgehenden Ansprüche, worin wenigstens einer der Substituenten $R^1$, $R^2$ und $R^3$ eine Methoxygruppe ist.

11. Verbindung nach einem der vorausgehenden Ansprüche, worin wenigstens einer der Substituenten $R^1$, $R^2$ und $R^3$ eine Acylamidogruppe ist.

12. Verbindung nach einem der vorausgehenden Ansprüche, worin wenigstens einer der Substituenten $R^1$, $R^2$ und $R^3$ eine Sulfonamidogruppe ist, wie sie in Anspruch 1 definiert ist.

13. Verbindung nach einem der vorausgehenden Ansprüche, worin wenigstens einer der Substituenten $R^1$, $R^2$ und $R^3$ eine Aminogruppe oder eine Alkylaminogruppe ist.

14. Verbindung nach einem der vorausgehenden Ansprüche, worin wenigstens einer der Substituenten $R^1$, $R^2$ und $R^3$ eine Nitrogruppe ist.

15. Verbindung nach einem der vorausgehenden Ansprüche, worin zwei der Substituenten $R^1$, $R^2$ und $R^3$ Wasserstoffatome bedeuten.

16. Verbindung nach Anspruch 15, worin zwei der Substituenten $R^1$, $R^2$ und $R^3$ ein Wasserstoffatom bedeuten und der andere eine Methoxygruppe, eine Acylamidogruppe oder eine Sulfonamidogruppe, wie sie in Anspruch 1 definiert ist bedeutet.

17. Verbindung nach Anspruch 4, worin $R^6$ ein Wasserstoffatom oder eine Alkylgruppe mit 1 bis 6 Kohlenstoffatomen bedeutet.

18. Verbindung nach einem der vorausgehenden Ansprüche für die Verwendung als ein antipsychotisches oder antiemetisches Mittel.

19. Pharmazeutisches Präparat, das als ein antipsychotisches oder antiemetisches Mittel brauchbar ist und einen pharmazeutisch verträglichen Träger in Kombination mit einer therapeutisch wirksamen Menge einer Verbindung nach einem der vorausgehenden Ansprüche umfaßt.

20. Verfahren zur Herstellung eines Benzofurancarboxamids der allgemeinen Formel

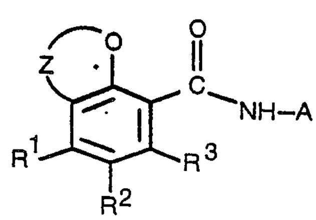

I

worin Z die erforderlichen Atome bedeutet, um einen Benzo[b]furanring ode einen Dihydrobenzo[b]-furanring zu vervollständigen, von denen jeder mit einer Alkylgruppe substituiert sein kann, $R^1$, $R^2$ und $R^3$ gleich oder verschieden sein können und jeweils ein Wasserstoffatom, eine Alkylgruppe mit 1 bis 6 Kohlenstoffatomen, ein Halogen-atom, eine Aminogruppe, eine Alkylaminogruppe, in welcher der oder die

Alkylsubstituenten 1 bis 6 Kohlenstoffatome enthalten, eine Alkoxygruppe mit 1 bis 6 Kohlenstoffatomen, eine Acylamidogruppe mit 1 bis 6 Kohlenstoffatomen, eine Sulfonamidogruppe mit bis zu 6 Kohlenstoffatomen, die an das Benzo[b]furansystem durch das N-Atom gebunden ist, oder eine Nitrogruppe bedeutet und A eine Tertiäraminoalkylgruppe mit der Atomfolge —C—C—N bedeutet, oder eines Säureadditionssalzes hiervon, bei dem man eine Benzo- oder Dihydrobenzo[b]furan-7-carbonsäure, deren Säurechlorid oder Ester der allgemeinen Formel

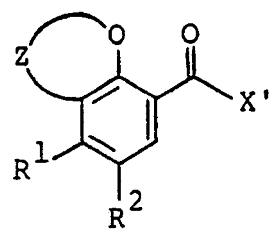

worin $R^1$, $R^2$ und Z wie oben definiert sind und $X^1$ ein Chloratom oder eine Hydroxyl- oder Alkoxygruppe bedeutet, mit einem Amin der allgemeinen Formel $A—NH_2$, worin A wie oben definiert ist, kondensiert.

**Patentansprüche für die Vertragsstaaten: CH DE FR GB NL**

1. Verbindung der Formel (I)

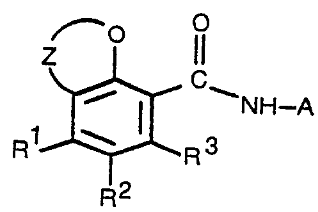

I

worin Z die erforderlichen Atome bedeutet, um einen Benzo[b]furanring oder einen Dihydrobenzo[b]furanring zu vervollständigen, von denen jeder mit einer Alkylgruppe substituiert sein kann, $R^1$, $R^2$ und $R^3$ gleich oder verschieden sein können und jeweils ein Wasserstoffatom, eine Alkylgruppe mit 1 bis 6 Kohlenstoffatomen, ein Halogenatom, eine Aminogruppe, eine Alkylaminogruppe, in welcher der oder die Alkylsubstituenten 1 bis 6 Kohlenstoffatome enthalten, eine Alkoxygruppe mit 1 bis 6 Kohlenstoffatomen, eine Acylamidogruppe mit 1 bis 6 Kohlenstoffatomen, eine Sulfonamidogruppe mit bis zu 6 Kohlenstoffatomen, die an das Benzo[b]furansystem durch das N-Atom gebunden ist, oder eine Nitrogruppe bedeutet und A eine Aminoalkylgruppe ist, die die Atomfolge —C—C—N enthält, und deren Säureadditionssalze.

2. Verbindung der Formel II

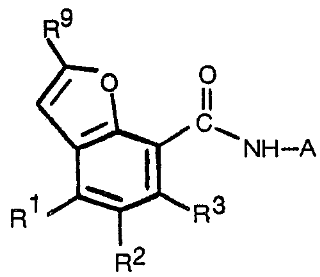

II

worin A, $R^1$, $R^2$ und $R^3$ wie in Anspruch 1 definiert sind und $R^9$ ein Wasserstoffatom oder eine Alkylgruppe, die 1 bis 3 Kohlenstoffatome enthält, bedeutet, und deren Säureadditionssalze.

3. Verbindung der Formel III

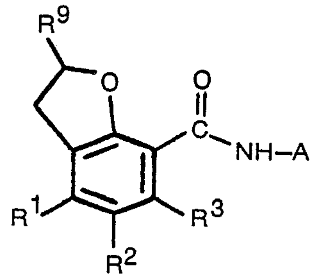

III

worin A, $R^1$, $R^2$ und $R^3$ wie in Anspruch 1 definiert sind und $R^9$ ein Wasserstoffatom oder eine Alkylgruppe, die 1 bis 3 Kohlenstoffatome enthält, bedeutet, und deren Säureadditionssalze.

4. Verbindung der Formel IV

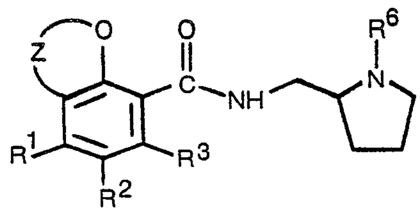

IV

worin Z die erforderlichen Atome bedeutet, um einen unsubstituierten oder alkylsubstituierten Benzo[b]-furan- oder Dihydrobenzo[b]furanring zu vervollständigen, und $R^1$, $R^2$ und $R^3$ wie in Anspruch 1 definiert sind und $R^6$ ein Wasserstoffatom oder eine Alkylgruppe mit 1 bis 6 Kohlenstoffatomen bedeutet, und deren Säureadditionssalze.

5. Verbindung nach einem der Ansprüche 1 bis 3, worin A einen der Substituenten

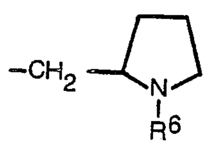

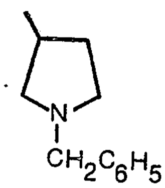

$-CH_2CH_2NR^7R^8$

bedeutet, worin $R^6$, $R^7$ und $R^8$ jeweils ein Wasserstoffatom oder eine Alkylgruppe mit 1 bis 6 Kohlenstoffatomen bedeutet.

6. Verbindung nach Anspruch 5, worin A eine 2-Pyrrolidinylmethylgruppe bedeutet.

7. Verbindung nach Anspruch 1 oder Anspruch 4, worin Z die erforderlichen Atome bedeutet, um einen 2-Methyl- oder 3-Methylbenzo[b]furanring zu vervollständigen.

8. Verbindung nach Anspruch 1 oder Anspruch 4, worin Z die erforderlichen Atome bedeutet, um einen 2-Methyl- oder 3-Methyl-2,3-dihydrobenzo[b]furanring zu vervollständigen.

9. Verbindung nach einem der vorausgehenden Ansprüche, worin wenigstens einer der Substituenten $R^1$, $R^2$ und $R^3$ ein Halogenatom ist.

10. Verbindung nach einem der vorausgehenden Ansprüche, worin wenigstens einer der Substituenten $R^1$, $R^2$ und $R^3$ eine Methoxygruppe ist.

11. Verbindung nach einem der vorausgehenden Ansprüche, worin wenigstens einer der Substituenten $R^1$, $R^2$ und $R^3$ eine Acylamidogruppe ist.

12. Verbindung nach einem der vorausgehenden Ansprüche, worin wenigstens einer der Substituenten $R^1$, $R^2$ und $R^3$ eine Sulfonamidogruppe ist, wie sie in Anspruch 1 definiert ist.

13. Verbindung nach einem der vorausgehenden Ansprüche, worin wenigstens einer der Substituenten $R^1$, $R^2$ und $R^3$ eine Aminogruppe oder eine Alkylaminogruppe ist.

14. Verbindung nach einem der vorausgehenden Ansprüche, worin wenigstens einer der Substituenten $R^1$, $R^2$ und $R^3$ eine Nitrogruppe ist.

15. Verbindung nach einem der vorausgehenden Ansprüche, worin zwei der Substituenten $R^1$, $R^2$ und $R^3$ Wasserstoffatome bedeuten.

16. Verbindung nach Anspruch 15, worin zwei der Substituenten $R^1$, $R^2$ und $R^3$ ein Wasserstoffatom bedeuten und der andere eine Methoxygruppe, eine Acylamidogruppe oder eine Sulfonamidogruppe, wie sie in Anspruch 1 definiert ist bedeutet.

17. Verbindung nach Anspruch 4, worin $R^6$ ein Wasserstoffatom oder eine Alkylgruppe mit 1 bis 6 Kohlenstoffatomen bedeutet.

18. Verbindung nach einem der vorausgehenden Ansprüche für die Verwendung als ein antipsychotisches oder antiemetisches Mittel.

19. Pharmazeutisches Präparat, das als ein antipsychotisches oder antiemetisches Mittel brauchbar ist und einen pharmazeutisch verträglichen Träger in Kombination mit einer therapeutisch wirksamen Menge einer Verbindung nach einem der vorausgehenden Ansprüche umfaßt.

20. Verfahren zur Herstellung eines Benzofurancarboxamids der allgemeinen Formel

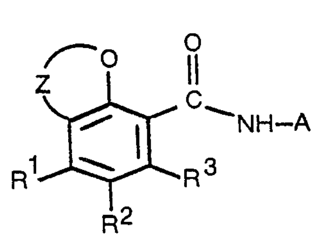

I

worin Z die erforderlichen Atome bedeutet, um einen Benzo[b]furanring oder einen Dihydrobenzo[b]-furanring zu vervollständigen, wobei jeder mit einer Alkylgruppe substituiert sein kann, $R^1$, $R^2$ und $R^3$ gleich oder verschieden sein können und jeweils ein Wasserstoffatom, eine Alkylgruppe mit 1 bis 6 Kohlenstoffatomen, ein Halogen-atom, eine Aminogruppe, eine Alkylaminogruppe, in welcher der oder die Alkylsubstituenten 1 bis 6 Kohelnstoffatome enthalten, eine Alkoxygruppe mit 1 bis 6 Kohlenstoffatomen, eine Acylamidogruppe mit 1 bis 6 Kohlenstoffatomen, eine Sulfonamidogruppe mit bis zu 6 Kohlenstoffatomen, die mit dem Benzo[b]furansystem durch das N-Atom gebunden ist, oder eine Nitrogruppe bedeutet und A eine Tertiäraminoalkylgruppe bedeutet, die die Atomfolge

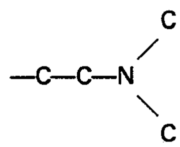

enthält, worin das Stickstoffatom Teil eines Pyrrolidinylringes bilden kann, oder eines Säureadditionssalzes hiervon, bei dem man ein Benzo- oder Dihydrobenzo[b]furan-7-carbonsäurechlorid oder einen Benzo- oder Dihydrobenzo[b]furan-7-carbonsäureester mit einem Amin kondensiert und ein Carboxamid in der Form seines Säuresalzes gewinnt.

**Patentansprüche für den Vertragsstaat: AT**

1. Verfahren zur Herstellung eines Benzofurancarboxamids der allgemeinen Formel

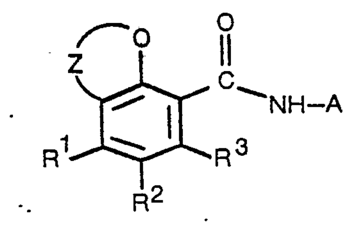

I

worin Z die erforderlichen Atome bedeutet, um einen Benzo[b]furanring ode einen Dihydrobenzo[b]-furanring zu vervollständigen, von denen jeder mit einer Alkylgruppe substituiert sein kann, $R^1$, $R^2$ und $R^3$ gleich oder verschieden sein können und jeweils ein Wasserstoffatom, eine Alkylgruppe mit 1 bis 6 Kohlenstoffatomen, ein Halogen-atom, eine Aminogruppe, eine Alkylaminogruppe, in welcher der oder die Alkylsubstituenten 1 bis 6 Kohlenstoffatome enthalten, eine Alkoxygruppe mit 1 bis 6 Kohlenstoffatomen, eine Acylamidogruppe mit 1 bis 6 Kohlenstoffatomen, eine Sulfonamidogruppe mit bis zu 6 Kohlenstoffatomen, die an das Benzo[b]furansystem durch das N-Atom gebunden ist, oder eine Nitrogruppe bedeutet und A eine Tertiäraminoalkylgruppe mit der Atomfolge —C—C—N bedeutet, oder eines Säureadditionssalzes hiervon, bei dem man eine Benzo- oder Dihydrobenzo[b]furan-7-carbonsäure, deren Säurechlorid oder Ester der allgemeinen Formel

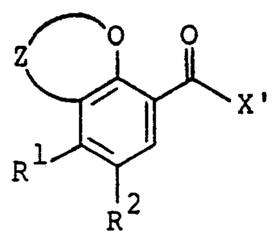

worin $R^1$, $R^2$ und Z wie oben definiert sind und $X^1$ ein Chloratom oder eine Hydroxyl- oder Alkoxygruppe bedeutet, mit einem Amin der allgemeinen Formel, $A—NH_2$ worin A wie oben definiert ist, kondensiert.

2. Verfahren nach Anspruch 1, worin die Carbonsäure, deren Säurechlorid oder Ester eine Verbindung der Formel

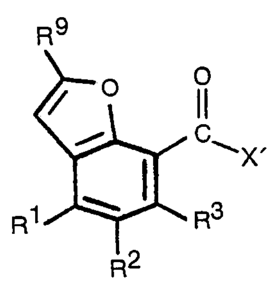

ist, worin $X^1$, $R^1$, $R^2$ und $R^3$ wie in Anspruch 1 definiert sind und $R^9$ ein Wasserstoffatom oder eine Alkylgruppe mit 1 bis 3 Kohlenstoffatomen bedeutet.

3. Verfahren nach Anspruch 1, bei dem die Carbonsäure, deren Säurechlorid oder Ester eine Verbindung der Formel

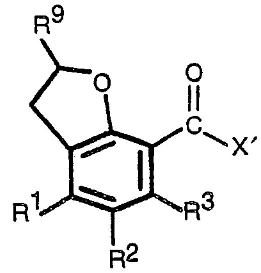

ist, worin $X^1$, $R^1$, $R^2$ und $R^3$ wie in Anspruch 1 definiert sind und $R^9$ ein Wasserstoffatom oder eine Alkylgruppe mit 1 bis 3 Kohlenstoffatomen bedeutet.

4. Verfahren nach einem der vorausgehenden Ansprüche, worin das Amin eine der folgenden Formeln hat:

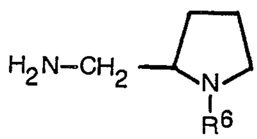

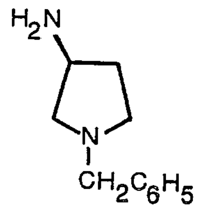

$H_2N-CH_2CH_2NR^7R^8$

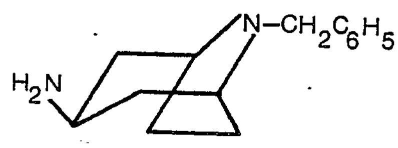

worin $R^6$, $R^7$ und $R^8$ jeweils ein Wasserstoffatom oder eine Alkylgruppe mit 1 bis 6 Kohlenstoffatomen bedeuten.

5. Verfahren nach Anspruch 4, worin A eine 2-Pyrrolidinylmethylgruppe bedeutet.

6. Verfahren nach Anspruch 1 oder Anspruch 4, worin Z die erforderlichen Atome bedeutet, um einen 2-Methyl- oder 3-Methylbenzo[b]furanring zu vervollständigen.

7. Verfahren nach Anspruch 1 oder Anspruch 4, worin Z die erforderlichen Atome bedeutet, um einen 2-Methyl- oder 3-Methyl-2,3-dihydrobenzo[b]furanring zu vervollständigen.

8. Verfahren nach einem der vorausgehenden Ansprüche, worin wenigstens einer der Substituenten $R^1$, $R^2$ und $R^3$ ein methoxygruppe ist.

9. Verfahren nach einem der vorausgehenden Ansprüche, worin der Carbonsäureester ein Methylester ist und durch:

Alkylieren der 2-Hydroxylgruppe eines substituierten oder unsubstituierten Methylsalicylates, das Gruppen $R^1$, $R^2$ in der 4- bzw. 5-Stellung hat, durch eine nucleophile Substitution mit einem eine Alkylhalogenid,

Unterziehen des so erhaltenen Zwischenproduktes einer Claisen-Umlagerung unter Bildung eines Methyl-(3-alkylsalicylats),

Oxidieren des Alkylsubstituenten zu einer Aldehyd- oder Ketongruppe und

Umsetzung der 2-Hydroxygruppe mit der Aldehyd- oder Ketongruppe unter Cyclisierung

erhalten wurde.

10. Verfahren nach Anspruch 9, bei dem die Cyclisierung in Gegenwart eines Säurekatalysators unter Bildung eines Benzo[b]furanringes durchgeführt wird.

11. Verfahren nach Anspruch 9, bei dem die Cyclisierung durch Behandlung des Methyl-(3-alkylsalicylates) mit Quecksilber-II-acetat und anschließend mit Natriumborhydrid unter Bildung eines Dihydrobenzo[b]furanringes durchgeführt wird.

12. Verfahren nach Anspruch 3, bei dem das Amin mit einer 2,3-Dihydrobenzo[b]furan-7-carbonsäure umgesetzt wird, die durch Lithiumbehandlung eines 2,3-Dihydro[b]furans mit n-Butyllithium und anschließendes Abschrecken mit Trockeneis und Ansäuern mit konzentrierter HCl hergestellt wurde.

13. Verfahren nach Anspruch 2, bei dem das Amin mit einer Benzo[b]furan-7-carbonsäure umgesetzt wird, die durch Alkylierung eines 2,6-Dibromphenols mit 1,2-Dibromethan unter Bildung eines Phenyloxyethylbromids, das dann mit n-Butyllithium behandelt, in Trockeneis abgeschreckt und angeäusert wird, hergestellt wurde.

**Revendications pour les Etats contractants: BE IT LU SE**

1. Composé ayant la formule (I)

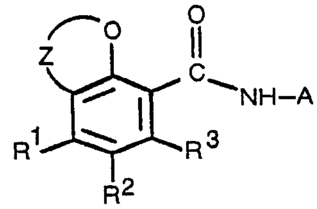

I

dans laquelle Z représente les atomes nécessaires pour compléter un anneau benzo-(b)-furane ou un anneau dihydrobenzo-(b)-furane, dont l'un peut être substitué avec un groupe alkyle, $R^1$, $R^2$ et $R^3$ peuvent être les mêmes ou différents, et chacun représente un atome hydrogène, un groupe alkyl ayant 1 à 6 atomes de carbone, un atome halogène, un groupe amino, un groupe alkylamino dans lequel le ou les substituants alkyl contiennent 1 à 6 atomes de carbone, un groupe alkoxy ayant 1 à 6 atomes de carbone, un groupe acylamido ayant 1 à 6 atomes de carbone, un groupe sulfonamido ayant jusqu'à 6 atomes de carbone et étant attaché au système benzo-(b)-furane par l'atome N, ou un groupe nitro; et A représente un groupe aminoalkyl contenant la séquence atomique —C—C—N, et ses sels d'addition acides.

2. Composé ayant la formule (II)

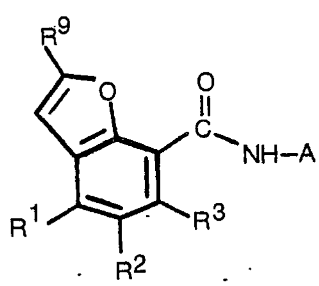

II

dans laquelle A, $R^1$, $R^2$ et $R^3$ sont comme définis dans la revendication 1, et $R^9$ représente un atome hydrogène ou un groupe alkyl contenant 1 à 3 atomes de carbone; et ses sels d'addition acides.

3. Composé ayant la formule (III)

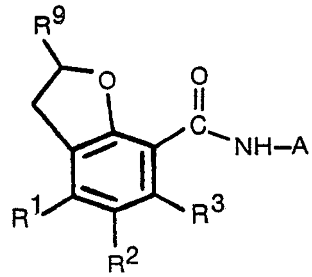

III

dans laquelle A, $R^1$, $R^2$ et $R^3$ sont comme définis dans la revendication 1, et $R^9$ représente un atome hydrogène ou un groupe alkyl contenant 1 à 3 atomes de carbone; et ses sels d'addition acides.

4. Composé ayant la formule (IV)

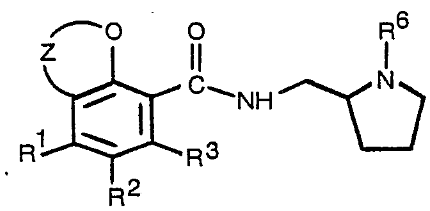

IV

dans laquelle Z représente les atomes nécessaires pour compléter un anneau benzo-(b) furane ou dihydrobenzo-(b)-furane, et $R^1$, $R^2$ et $R^3$ sont comme définis dans la revendication 1, et $R^6$ représente un atome d'hydrogène ou un groupe alkyl ayant 1 à 6 atomes de carbone; et ses sels d'addition acides.

5. Composé selon une des revendications 1 à 3, dans lequel A représente un substituant choisi parmi

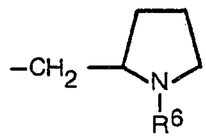

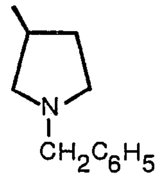

$-CH_2CH_2NR^7R^8$

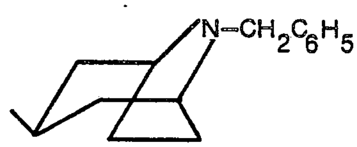

où $R^6$, $R^7$ et $R^8$ représentent chacun un atome hydrogène ou un groupe alyle contenant 1 à 6 atomes de carbone.

6. Composé selon la revendication 5, dans lequel A représente un groupe 2-pyrrolidinylméthyle.

7. Composé selon la revendication 1 ou la revendication 4, dans lequel Z représente les atomes nécessaires pour compléter un anneau 2-méthyl ou 3-methyl benzo-(b)-furane.

8. Composé selon la revendication 1 ou la revendication 4, dans lequel Z représente les atomes nécessaires pour compléter un anneau 2-méthyl ou 3-methyl 2,3-dihydrobenzo-(b)-furane.

9. Composé selon l'une quelconque des revendications précédentes dans lequel au moins un des substituants $R^1$, $R^2$ et $R^3$ est un atome halogène.

10. Composé selon l'une quelconque des revendications précédentes dans lequel au moins un des substituants $R^1$, $R^2$ et $R^3$ est un groupe méthoxy.

11. Composé selon l'une quelconque des revendications précédentes dans lequel au moins un des substituants $R^1$, $R^2$ et $R^3$ est un groupe acylamido.

12. Composé selon l'une quelconque des revendications précédentes dans lequel au moins un des substituants $R^1$, $R^2$ et $R^3$ est un groupe sulfonamido tel que défini dans la revendication 1.

13. Composé selon l'une quelconque des revendications précédentes dans lequel au moins un des substituants $R^1$, $R^2$ et $R^3$ est un groupe amino ou un groupe alkylamino.

14. Composé selon l'une quelconque des revendications précédentes dans lequel au moins un des substituants $R^1$, $R^2$ et $R^3$ est un groupe nitro.

15. Composé selon l'une quelconque des revendications précédentes dans lequel deux des substituants $R^1$, $R^2$ et $R^3$ représentent des atomes d'hydrogène.

16. Composé selon la revendication 15, dans lequel deux des substituants $R^1$, $R^2$ et $R^3$ représentent un atome d'hydrogène et l'autre est un groupe méthoxy, un groupe acylamido ou un groupe sulfonamido tel que défini dans la revendication 1.

17. Composé selon la revendication 4, dans lequel $R^6$ représente un atome d'hydrogène ou un groupe alkyl ayant un à six atomes de carbone.

18. Composé selon une quelconque des revendications précédentes utilisable comme agent antipsychotique ou antiémétique.

19. Préparation pharmaceutique utilisable comme agent antipsychotique ou antiémétique comprenant un support pharmaceutiquement acceptable en combinaison avec une quantité thérapeutiquement efficace d'un composé selon une quelconque des revendications précédentes.

20. Procédé pour produire benzofuranecarboxamide ayant une formule générale:

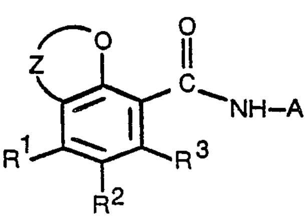

I

dans laquelle Z représente les atomes nécessaires pour compléter un anneau benzo-(b)-furane ou un anneau dihydrobenzo-(b)-furane, dont l'un peut être substitué avec un groupe alkyle, $R^1$, $R^2$ et $R^3$ peuvent être les mêmes ou différents, et chacun représente un atome hydrogène, un groupe alkyl ayant 1 à 6 atomes de carbone, un atome halogène, un groupe amino, un groupe alkylamino dans lequel le ou les substituants alkyl contiennent 1 à 6 atomes de carbone, un groupe alkoxy ayant 1 à 6 atomes de carbone,

un groupe acylamido ayant 1 à 6 atomes de carbone, un groupe sulfonamido ayant jusqu'à 6 atomes de carbone et étant attaché au système benzo-(b)-furane par l'atome N, ou un groupe nitro; et A représente un groupe aminoalkyl tertiaire contenant la séquence atomique —C—C—N, ou un de ses sels d'addition acides, qui comprend l'étape de condenser un acide benzo- ou dihydrobenzo-(b)-furane-7-carboxylique, un ester ou un chlorure acide ayant la formule générale:

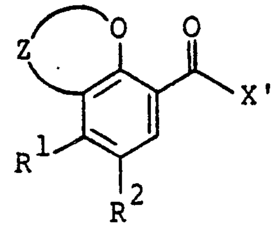

dans laquelle $R^1$, $R^2$ et Z sont comme définis ci-dessus et $X^1$ représente un atome de chlore ou un groupe hydroxyle ou alkoxy, avec une amine ayant la formule générale A—$NH_2$, dans laquelle A est comme défini ci-dessus.

**Revendications pour les Etats contractants: CH DE FR GB NL**

1. Composé ayant la formule (I)

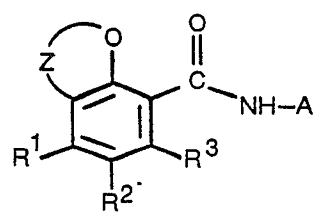

I

dans laquelle Z représente les atomes nécessaires pour compléter un anneau benzo-(b)-furane ou un anneau dihydrobenzo-(b)-furane, dont l'un peut être substitué avec un groupe alkyle, $R^1$, $R^2$ et $R^3$ peuvent être les mêmes ou différents, et chacun représente un atome hydrogène, un groupe alkyl ayant 1 à 6 atomes de carbone, un atome halogène, un groupe amino, un groupe alkylamino dans lequel le ou les substituants alkyl contiennent 1 à 6 atomes de carbone, un groupe alkoxy ayant 1 à 6 atomes de carbone, un groupe acylamido ayant 1 à 6 atomes de carbone, un groupe sulfonamido ayant jusqu'à 6 atomes de carbone et étant attaché au système benzo-(b)-furane par l'atome N, ou un groupe nitro; et A représente un groupe aminoalkyl tertiaïre contenant la séquence atomique —C—C—N, et dans laquelle l'atome d'azote peut faire partie d'un anneau pyrrolidinyl et ses sels d'addition acides.

2. Composé ayant la formule (II)

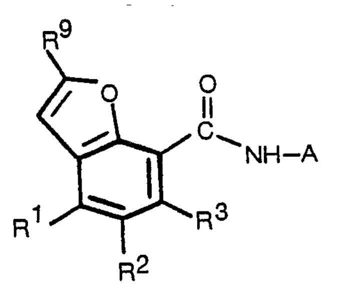

II

dans laquelle A, $R^1$, $R^2$ et $R^3$ sont comme définis dans la revendication 1, et $R^9$ représente un atome hydrogène ou un groupe alkyl contenant 1 à 3 atomes de carbone; et ses sels d'addition acides.

3. Composé ayant la formule (III)

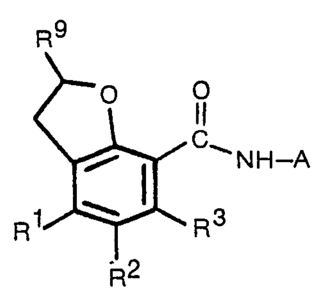

III

dans laquelle A, $R^1$, $R^2$ et $R^3$ sont comme définis dans la revendication 1, et $R^9$ représente un atome hydrogène ou un groupe alkyl contenant 1 à 3 atomes de carbone; et ses sels d'addition acides.

4. Composé ayant la formule (IV)

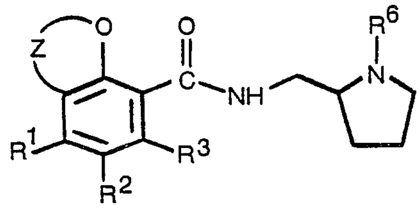

IV

dans laquelle Z représente les atomes nécessaires pour compléter un anneau benzo-(b) furane ou dihydrobenzo-(b)-furane, et $R^1$, $R^2$ et $R^3$ sont comme définis dans la revendication 1, et $R^6$ représente un atome d'hydrogène ou un groupe alkyl ayant 1 à 6 atomes de carbone; et ses sels d'addition acides.

5. Composé selon une des revendications 1 à 3, dans lequel A représente un substituant choisi parmi

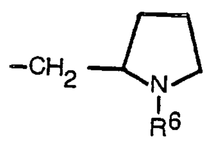

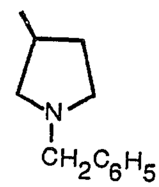

$-CH_2CH_2NR^7R^8$

où $R^6$, $R^7$ et $R^8$ représentent chacun un atome hydrogène ou un groupe alyle contenant 1 à 6 atomes de carbone.

6. Composé selon la revendication dans lequel A représente un groupe 2-pyrrolidinylméthyle.

7. Composé selon la revendication 1 ou la revendication 4, dans lequel Z représente les atomes nécessaires pour compléter un anneau 2-méthyl ou 3-methyl benzo-(b)-furane.

8. Composé selon la revendication 1 ou la revendication 4, dans lequel Z représente les atomes nécessaires pour compléter un anneau 2-méthyl ou 3-methyl 2,3-dihydrobenzo-(b)-furane.

9. Composé selon l'une quelconque des revendications précédentes dans lequel au moins un des substituants $R^1$, $R^2$ et $R^3$ est un atome halogène.

10. Composé selon l'une quelconque des revendications précédentes dans lequel au moins un des substituants $R^1$, $R^2$ et $R^3$ est un groupe méthoxy.

11. Composé selon l'une quelconque des revendications précédentes dans lequel au moins un des substituants $R^1$, $R^2$ et $R^3$ est un groupe acylamido.

12. Composé selon l'une quelconque des revendications précédentes dans lequel au moins un des substituants $R^1$, $R^2$ et $R^3$ est un groupe sulfonamido tel que défini dans la revendication 1.

13. Composé selon l'une quelconque des revendications précédentes dans lequel au moins un des substituants $R^1$, $R^2$ et $R^3$ est un groupe amino ou un groupe alkylamino.

14. Composé selon l'une quelconque des revendications précédentes dans lequel au moins un des substituants $R^1$, $R^2$ et $R^3$ est un groupe nitro.

15. Composé selon l'une quelconque des revendications précédentes dans lequel deux des substituants $R^1$, $R^2$ et $R^3$ représentent des atomes d'hydrogène.

16. Composé selon la revendication 15, dans lequel deux des substituants $R^1$, $R^2$ et $R^3$ représentent un atome d'hydrogène et l'autre est un groupe méthoxy, un groupe acylamido ou un groupe sulfonamido tel que défini dans la revendication 1.

17. Composé selon la revendication 4, dans lequel $R^6$ représente un atome d'hydrogène ou un groupe alkyl ayant un à six atomes de carbone.

18. Composé selon une quelconque des revendications précédentes utilisable comme agent antipsychotique ou antiémétique.

19. Préparation pharmaceutique utilisable comme agent antipsychotique ou antiémétique comprenant un support pharmaceutiquement acceptable en combinaison avec une quantité thérapeutiquement efficace d'un composé selon une quelconque des revendications précédentes.

20. Procédé pour produire benzofuranecarboxamide ayant une formule générale:

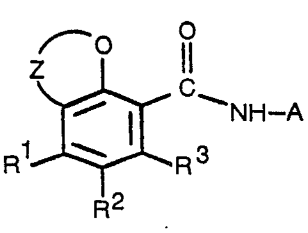

I

dans laquelle Z représente les atomes nécessaires pour compléter un anneau benzo-(b)-furane ou un anneau dihydrobenzo-(b)-furane, dont l'un peut être substitué avec un groupe alkyle, $R^1$, $R^2$ et $R^3$ peuvent être les mêmes ou différents, et chacun représente un atome hydrogène, un groupe alkyl ayant 1 à 6 atomes de carbone, un atome halogène, un groupe amino, un groupe alkylamino dans lequel le ou les substituants alkyl contiennent 1 à 6 atomes de carbone, un groupe alkoxy ayant 1 à 6 atomes de carbone, un groupe acylamido ayant 1 à 6 atomes de carbone, un groupe sulfonamido ayant jusqu'à 6 atomes de carbone et étant attaché au système benzo-(b)-furane par l'atome N, ou un groupe nitro; et A représente un groupe aminoalkyl tertiaire contenant la séquence atomique —C—C—N dans laquelle l'atome d'azote peut faire partie d'un anneau de pyrrolidinyl ou un de ses sels d'addition, qui comporte la condensation d'un ester ou chlorure acide benzo- ou dihydrobenzo-(b)-furane-7-carboxylique avec une amine et la récupération d'une carboxamide sous forme de son sel acide.

**Revendications por l'Etat contractant: AT**

1. Procédé pour produire un benzofuranecarboxamide ayant une formule générale:

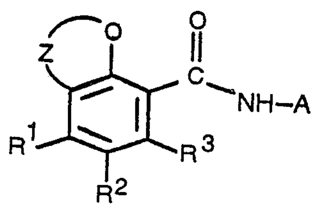

I

dans laquelle Z représente les atomes nécessaires pour compléter un anneau benzo-(b)-furane ou un anneau dihydrobenzo-(b)-furane, dont l'un peut être substitué avec un groupe alkyle, $R^1$, $R^2$ et $R^3$ peuvent être les mêmes ou différents, et chacun représente un atome hydrogène, un groupe alkyl ayant 1 à 6 atomes de carbone, un atome halogène, un groupe amino, un groupe alkylamino dans lequel le ou les substituants alkyl contiennent 1 à 6 atomes de carbone, un groupe alkoxy ayant 1 à 6 atomes de carbone, un groupe acylamido ayant 1 à 6 atomes de carbone, un groupe sulfonamido ayant jusqu'à 6 atomes de carbone et étant attaché au système benzo-(b)-furane par l'atome N, ou un groupe nitro; et A représente un groupe aminoalkyl tertiaire contenant la séquence atomique —C—C—N, ou un de ses sels d'addition acides, qui comprend l'étape de condenser un acide benzo- ou dihydrobenzo-(b)-furane-7-carboxylique, un ester ou un chlorure acide ayant la formule générale:

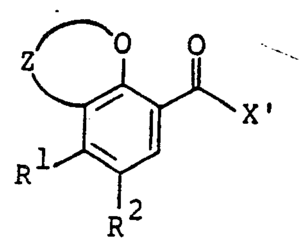

dans laquelle $R^1$, $R^2$ et Z sont comme définis et $X^1$ représente un atome de chlore ou un groupe hydroxyle ou alkoxy, avec une amine ayant la formule générale $A—NH_2$, dans laquelle A est comme défini ci-dessus.

2. Procédé selon la revendication 1, dans lequel ledit acide carboxylique, l'ester ou le chlorure acide est un composé de la formule:

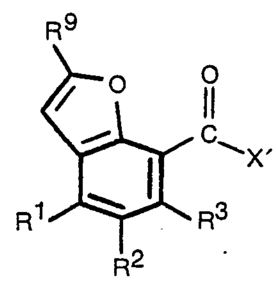

dans laquelle $X^1$, $R^1$, $R^2$ et $R^3$ sont comme définis dans la revendication 1, et représente un atome d'hydrogène ou un groupe alkyl contenant 1 à 3 atomes de carbone.

3. Procédé selon la revendication 1, dans lequel letid acide carboxylique, l'ester ou le chlorure acide est un composé de la formule:

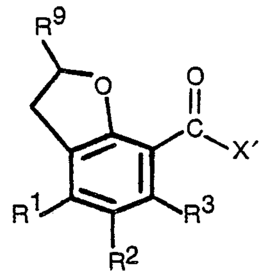

dans laquelle $X^1$, $R^1$, $R^2$ et $R^3$ sont comme définis dans la revendication 1, et $R^9$ représente un atome d'hydrogène ou un groupe alkyl contenant 1 à 3 atomes de carbone.

4. Procédé selon une quelconque des revendications précédentes dans lequel l'amine a une des formules suivantes:

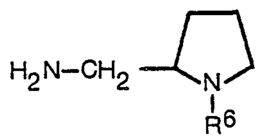

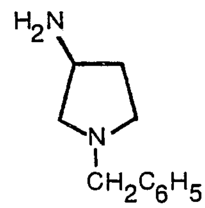

$H_2N-CH_2CH_2NR^7R^8$

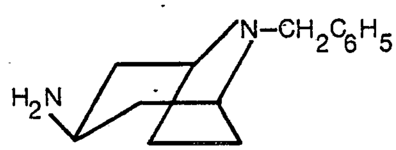

où $R^6$, $R^7$, $R^8$ représentent chacun un atome hydrogène ou un groupe alkyl contenant 1 à 6 atomes de carbone.

5. Procédé selon la revendication 4 dans lequel A représente un groupe 2-pyrrolidinylméthyle.

6. Procédé selon la revendication 1 ou la revendication 4 dans lequel Z représente les atomes nécessaires pour compléter un anneau 2-méthyl ou 3-methyl benzo-(b)-furane.

7. Procédé selon la revendication 1 ou la revendication 4 dans lequel Z représente les atomes nécessaires pour compléter un anneau 2-méthyl ou 3-methyl 2,3-dihydrobenzo-(b)-furane.

8. Procédé selon une quelconque des revendications précédents, dans lequel au moins un des substituants $R^1$, $R^2$ et $R^3$ est un grope méthoxy.

9. Procédé selon une quelconque des revendications précédentes, dans lequel ledit ester acide carboxylique est un ester méthyl et est obtenu par:

alkylation du groupe 2-hydroxyl de méthyl salicylate substitué ou non substitué, ayant des groupe $R^1$, $R^2$ aux positions 4- et 5- respectivement par substitution nucléophilique avec un halogénure alkyl,

soumettre le composé intermédiaire ainsi obtenu à un réarrangement claisien pour donner un 3-alkyl salicylate de méthyle,

oxyder le substituant alkyl en un groupe aldehyde ou cétone, et

faire réagir le groupe 2-hydroxy avec le groupe aldehyde ou cétone pour effectuer une cyclisation.

10. Procédé selon la revendication 9, dans lequel la cyclisation est effectuée en présence d'un catalyseur acide pour produire un anneau benzo-(b)-furane.

11. Procédé selon la revendication 9, dans lequel la cyclisation est effectuée en traitant le 3-alkyl salicyate de méthyle avec de l'acétate mercurique suivi par du borohydrure de sodium pour donner un anneau dihydrobenzo-(b)-furane.

12. Procédé selon la revendication 3, dans lequel on fait réagir l'amine avec un acide 2,3-dihydrobenzo-(b)-furane-7-carboxylique produit par lithiation de 2,3-dihydrobenzo-(b)-furane avec un n-butyl lithium, suivi par trempage avec de la glace sèche et acidification avec HCl concentré.

13. Procédé selon la revendication 2, dans lequel on fait réagir l'amine avec un acide benzo-(b)-furane-7-carboxylique produit par alkylation d'un 2,6-dibromophénol avec du 1,2-dibromoéthane pour produire un bromure phényloxyéthyle qui est ensuite lithié avec du n-butyl lithium trempé dans la glace sèche et acidifié.